# EUROPEAN PATENT SPECIFICATION

(11) **EP 1 597 370 B1**
(45) Date of publication and mention of the grant of the patent: **28.06.2006**
(21) Application number: 04709218.4
(22) Date of filing: 09.02.2004
(51) Int. Cl.: C12N 15/63, C12N 15/861, C12N 1/12, C07K 14/705, C12N 5/10, A61K 48/00

(54) **RHESUS CARCINO EMBRYONIC ANTIGEN, NUCLEOTIDES ENCODING SAME, AND USES THEREOF**
RHESUS-KARZINOEMBRYONALES ANTIGEN, DIESES CODIERENDE NUKLEOTIDE UND VERWENDUNGEN DAVON
ANTIGENE CARCINO-EMBRYONNAIRE RHESUS, NUCLEOTIDES CODANT POUR CET ANTIGENE ET UTILISATIONS ASSOCIEES

(30) Priority: 13.02.2003 US 447203 P
(43) Date of publication of application: 23.11.2005
(73) Proprietor: ISTITUTO DI RICERCHE DI BIOLOGIA MOLECOLARE P. ANGELETTI S.P.A., 00040 Pomezia (IT)
(72) Inventor: AURISICCHIO, L.; c/o Irbm, I-00040 Pomezia (IT); CILIBERTO, Gennaro; c/o Irbm, I-00040 Pomezia (IT); LAHM, Armin; c/o Irbm, I-00040 Pomezia (IT); LA MONICA, Nicola; c/o Irbm, I-00040 Pomezia (IT); MONACI, P.; c/o Irbm, I-00040 Pomezia (IT); PALOMBO, Fabio; c/o Irbm, I-00040 Pomezia (IT)
(74) Representative: Buchan, Gavin MacNicol
(86) International application number: PCT/EP2004/001181
(87) International publication number: WO 2004/072287

(56) References cited:
- WO-A-01/30382
- US-A- 5 274 087
- WHEELER C W ET AL: "Safety and immunogenicity of a recombinant vaccinia virus vaccine expressing a 70kDa fragment of the human carcinoembryonic antigen in a nonhuman primate" PROCEEDINGS OF THE AMERICAN ASSOCIATION FOR CANCER RESEARCH ANNUAL, vol. 36, no. 0, 1995, page 366 XP001181916 Eighty-sixth Annual Meeting of the American Association for Cancer Research;Toronto, Ontario, Canada; March 18-22, 1995, 1995 ISSN: 0197-016X
- IRVINE K ET AL: "CHARACTERIZATION OF IMMUNOLOGICAL RESPONSES USING A CEA RECOMBINANT VACCINIA VIRUS IN NON-HUMAN PRIMATES" PROCEEDINGS OF THE AMERICAN ASSOCIATION FOR CANCER RESEARCH ANNUAL, vol. 33, 1992, page 334 XP001181917 83RD ANNUAL MEETING OF THE AMERICAN ASSOCIATION FOR CANCER RESEARCH, SAN DIEGO, CALIFORNIA, USA, MAY, 1992 ISSN: 0197-016X
- KANTOR J ET AL: "Immunogenicity and safety of a recombinant vaccinia virus vaccine expressing the carcinoembryonic antigen gene in a nonhuman primate" CANCER RESEARCH, vol. 52, no. 24, 1992, pages 6917-6925, XP000605381 ISSN: 0008-5472
- JANTSCHEFF P ET AL: "SEARCH FOR CARCINOEMBRYONIC ANTIGEN-LIKE MOLECULES IN POLYMORPHONUCLEAR LEUKOCYTES OF NON-HUMAN PRIMATES USING MONOCLONAL ANTIBODIES" ARCHIV FUER GESCHWULSTFORSCHUNG, vol. 56, no. 2, 1986, pages 113-116, XP001181929 ISSN: 0003-911X

## Description

### FIELD OF THE INVENTION

The present invention relates generally to the therapy of cancer. More specifically, the present invention relates to the rhesus monkey homologue of the tumor associated polypeptide carcinoembryonic antigen, herein designated rhCEA, to isolated nucleic acid molecules which encode this protein, and to recombinant vectors and host cells comprising DNA encoding this protein. This invention also relates to adenoviral vector constructs carrying rhCEA and to their use in vaccines and pharmaceutical compositions for preventing and treating cancer.

### BACKGROUND OF THE INVENTION

The immunoglobulin superfamily (IgSF) consists of numerous genes that code for proteins with diverse functions, one of which is intercellular adhesion. IgSF proteins contain at least one Ig-related domain that is important for maintaining proper intermolecular binding interactions. Because such interactions are necessary to the diverse biological functions of the IgSF members, disruption or aberrant expression of many IgSF adhesion molecules has been correlated with many human diseases.

The carcinoembryonic antigen (CEA) belongs to a subfamily of the Ig superfamily consisting of cell surface glycoproteins. Members of the CEA subfamily are known as CEA-related cell adhesion molecules (CEACAMs). In recent scientific literature, the CEA gene has been renamed CEACAM5, although the nomenclature for the protein remains CEA. Functionally, CEACAMs have been shown to act as both homotypic and heterotypic intercellular adhesion molecules (Benchimol et al., *Cell* 57: 327-334 (1989)). In addition to cell adhesion, CEA inhibits cell death resulting from detachment of cells from the extracellular matrix and can contribute to cellular transformation associated with certain proto-oncogenes such as *Bcl2* and *C-Myc* (*see* Berinstein, *J. Clin Oncol.* 20(8): 2197-2207 (2002)).

Normal expression of CEA has been detected during fetal development and in adult colonic mucosa. CEA overexpression was first detected in human colon tumors over thirty years ago (Gold and Freedman, *J. Exp. Med.* 121:439-462 (1965)) and has since been found in nearly all colorectal tumors. Additionally, CEA overexpression is detectable in a high percentage of adenocarcinomas of the pancreas, breast and lung. Because of the prevalence of CEA expression in these tumor types, CEA is widely used clinically in the management and prognosis of these cancers.

The correlation between CEA expression and metastatic growth has also led to its identification as a target for molecular and immunological intervention for colorectal cancer treatment. One therapeutic approach targeting CEA is the use of anti-CEA antibodies (*see* Chester et al., *Cancer Chemother. Pharmacol.* 46 (Suppl): S8-S12 (2000)), while another is to activate the immune system to attack CEA-expressing tumors using CEA-based vaccines (for review, see Berinstein, *supra).*

Sequences coding for human CEA have been cloned and characterized (U.S. Patent No. 5,274,087; U.S. Patent No 5,571,710; and U.S. Patent No 5,843,761. See also Beauchemin et al., *Mol. Cell. Biol.* 7:3221-3230 (1987); Zimmerman et al., *Proc. Natl. Acad. Sci. USA* 84:920-924 (1987); Thompson et al. *Proc. Natl. Acad. Sci. USA* 84(9):2965-69 (1987)). Despite the isolation and identification of these CEA genes, it would be desirable to identify additional mammalian genes encoding CEA to allow for the development of a cancer vaccine which is efficacious and not hindered by self-tolerance.

### SUMMARY OF THE INVENTION

The present invention relates to isolated or purified nucleic acid molecules (polynucleotides) comprising a sequence of nucleotides that encode a novel rhesus monkey carcino embryonic antigen (hereinafter rhCEA) as set forth in SEQ ID NO:2 and SEQ ID NO:8. The DNA molecules disclosed herein may be transfected into a host cell of choice wherein the recombinant host cell provides a source for substantial levels of an expressed functional rhCEA protein (SEQ ID NO:2 and SEQ ID NO:8).

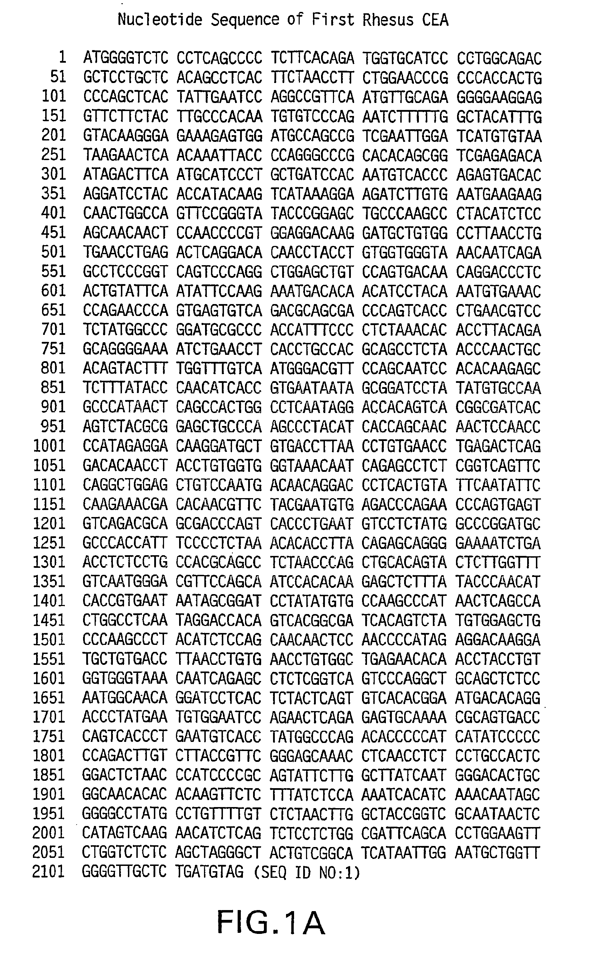

The present invention further relates to an isolated nucleic acid molecule which encodes mRNA that expresses a novel rhesus monkey CEA protein; this DNA molecule comprising the nucleotide sequence disclosed herein as SEQ ID NO:1. Nucleotide sequences coding for rhesus CEA are herein designated rhCEACAM5. A preferred aspect of this portion of the present invention is disclosed in FIGURE 1A, which shows a DNA molecule (SEQ ID NO:1) that encodes a novel rhCEA protein (SEQ ID NO:2).

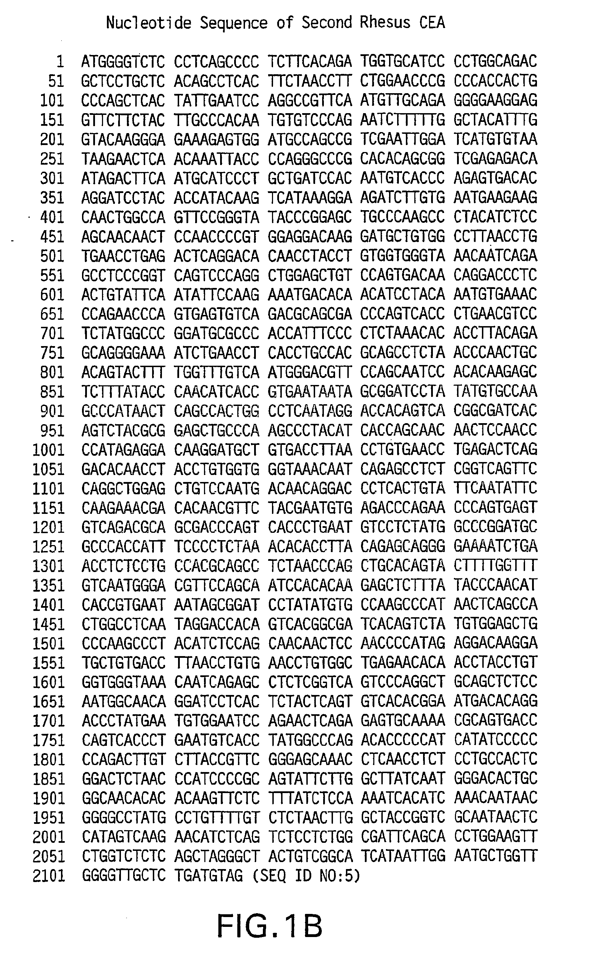

Another aspect of this invention is an isolated nucleic acid molecule which encodes a novel rhesus monkey CEA protein (SEQ ID NO:8), said nucleic acid molecule comprising a sequence of nucleotides as shown in FIGURE 1B and as set forth in SEQ ID NO:5.

The present invention also relates to recombinant vectors and recombinant host cells, both prokaryotic and eukaryotic, which contain the nucleic acid molecules disclosed throughout this specification.

The present invention further relates to a process for expressing a rhesus monkey CEA protein in a recombinant host cell, comprising: (a) introducing a vector comprising a nucleic acid as set forth in SEQ ID NO: 1 or SEQ ID NO:5 into a suitable host cell; and, (b) culturing the host cell under conditions which allow expression of said rhesus monkey CEA protein.

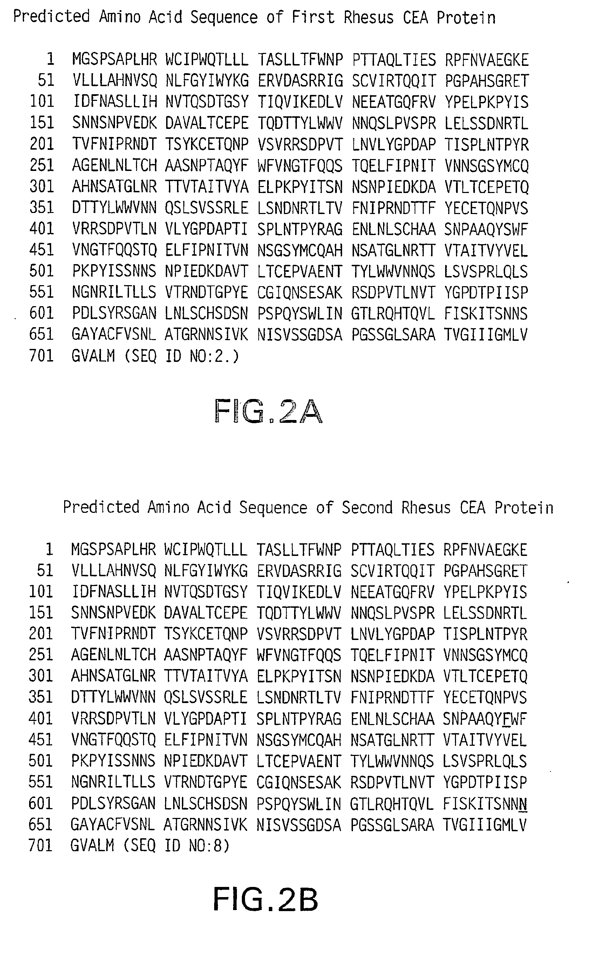

A preferred aspect of the present invention is a substantially purified form of a rhesus monkey CEA protein which consists of the amino acid sequence disclosed in FIGURE 2A (SEQ ID NO:2).

Another preferred aspect of the present invention is a substantially purified form of a rhesus monkey CEA protein which consists of the amino acid sequence disclosed in FIGURE 2B (SEQ ID NO:8).

Another preferred aspect of the present invention relates to a substantially purified, fully processed (including proteolytic processing, glycosylation and/or phosphorylation), mature rhCEA protein obtained from a recombinant host cell containing a DNA expression vector comprising nucleotide sequence as set forth in SEQ ID NO:1 or SEQ ID NO:5, which express the rhCEA protein. It is especially preferred that the recombinant host cell be a eukaryotic host cell, such as a mammalian cell line.

Yet another aspect of this invention is a method of preventing or treating cancer comprising administering to a mammal a vaccine vector comprising an isolated nucleic acid molecule, the isolated nucleic acid molecule comprising a sequence of nucleotides that encodes a rhesus monkey carcinoembryonic antigen (rhCEA) protein as set forth in SEQ ID NO:2 or SEQ ID NO:8.

The present invention further relates to an adenovirus vaccine vector comprising an adenoviral genome with a deletion in the E1 region, and an insert in the E1 region, wherein the insert comprises an expression cassette comprising: (a) a polynucleotide encoding a rhesus monkey CEA protein; and (b) a promoter operably linked to the polynucleotide.

The present invention also relates to a vaccine plasmid comprising a plasmid portion and an expression cassette portion, the expression cassette portion comprising: (a) a polynucleotide encoding a rhesus monkey CEA protein; and (b) a promoter operably linked to the polynucleotide.

Another aspect of the present invention is a method of protecting or a mammal from cancer or treating a mammal suffering from cancer comprising: (a) introducing into the mammal a first vector comprising: i) a polynucleotide encoding a rhesus monkey CEA protein; and ii) a promoter operably linked to the polynucleotide; (b) allowing a predetermined amount of time to pass; and (c) introducing into the mammal a second vector comprising: i) a polynucleotide encoding a rhesus monkey CEA protein; and ii) a promoter operably linked to the polynucleotide.

As used throughout the specification and in the appended claims, the singular forms "a," "an," and "the" include the plural reference unless the context clearly dictates otherwise.

As used throughout the specification and appended claims, the following definitions and abbreviations apply:

The term "promoter" refers to a recognition site on a DNA strand to which the RNA polymerase binds. The promoter forms an initiation complex with RNA polymerase to initiate and drive transcriptional activity. The complex can be modified by activating sequences termed "enhancers" or inhibiting sequences termed "silencers".

The term "cassette" refers to the sequence of the present invention that contains the nucleic acid sequence which is to be expressed. The cassette is similar in concept to a cassette tape; each cassette has its own sequence. Thus by interchanging the cassette, the vector will express a different sequence. Because of the restriction sites at the 5' and 3' ends, the cassette can be easily inserted, removed or replaced with another cassette.

The term "vector" refers to some means by which DNA fragments can be introduced into a host organism or host tissue. There are various types of vectors including plasmid, virus (including adenovirus), bacteriophages and cosmids.

The term "first generation," as used in reference to adenoviral vectors, describes said adenoviral vectors that are replication-defective. First generation adenovirus vectors typically have a deleted or inactivated E1 gene region, and preferably have a deleted or inactivated E3 gene region.

The designation "pV1J-rhCEA" refers to a plasmid construct disclosed herein comprising the human CMV immediate-early (IE) promoter with intron A, a full-length rhesus CEA gene, bovine growth hormone-derived polyadenylation and transcriptional termination sequences, and a minimal pUC backbone.

The designations "pMRK-Ad5-rhCEA" and "MRK-rhCEA" refer to a construct, disclosed herein, which comprises an Ad5 adenoviral genome deleted of the E1 and E3 regions. In this plasmid, the E1 region is replaced by a rhesus CEA gene in an E1 parallel orientation under the control of a human CMV promoter without intron A, followed by a bovine growth hormone polyadenylation signal.

The designation "pBS-rhCEA" refers to a construct disclosed herein comprising the pBluescriptII KS (+) plasmid and a full-length rhCEA gene.

The term "effective amount" means sufficient vaccine composition is introduced to produce the adequate levels of the polypeptide, so that an immune response results. One skilled in the art recognizes that this level may vary.

"Substantially free from other nucleic acids" means at least 90%, preferably 95%, more preferably 99%, and even more preferably 99.9%, free of other nucleic acids. As used interchangeably, the terms "substantially free from other nucleic acids," "substantially purified," "isolated nucleic acid" or "purified nucleic acid" also refer to DNA molecules which comprise a coding region for a rhesus CEA protein that has been purified away from other cellular components. Thus, a rhesus CEA DNA preparation that is substantially free from other nucleic acids will contain, as a percent of its total nucleic acid, no more than 10%, preferably no more than 5%, more preferably no more than 1%, and even more preferably no more than 0.1%, of non-rhesus CEA nucleic acids. Whether a given rhesus CEA DNA preparation is substantially free from other nucleic acids can be determined by such conventional techniques of assessing nucleic acid purity as, *e.g.,* agarose gel electrophoresis combined with appropriate staining methods, *e.g.*, ethidium bromide staining, or by sequencing.

"Substantially free from other proteins" or "substantially purified" means at least 90%, preferably 95%, more preferably 99%, and even more preferably 99.9%, free of other proteins. Thus, a rhesus monkey CEA protein preparation that is substantially free from other proteins will contain, as a percent of its total protein, no more than 10%, preferably no more than 5%, more preferably no more than 1%, and even more preferably no more than 0.1%, of non-rhesus monkey CEA proteins. Whether a given rhesus monkey CEA protein preparation is substantially free from other proteins can be determined by such conventional techniques of assessing protein purity as, *e.g.*, sodium dodecyl sulfate polyacrylamide gel electrophoresis (SDS-PAGE) combined with appropriate detection methods, *e.g.*, silver staining or immunoblotting.

As used interchangeably, the terms "substantially free from other proteins" or "substantially purified," or "isolated rhesus monkey CEA protein" or "purified rhesus monkey CEA protein" also refer to rhesus monkey CEA protein that has been isolated from a natural source. Use of the term "isolated" or "purified" indicates that rhesus monkey CEA protein has been removed from its normal cellular environment. Thus, an isolated rhesus monkey CEA protein may be in a cell-free solution or placed in a different cellular environment from that in which it occurs naturally. The term isolated does not imply that an isolated rhesus monkey CEA protein is the only protein present, but instead means that an isolated rhCEA protein is substantially free of other proteins and non-amino acid material (e.g., nucleic acids, lipids, carbohydrates) naturally associated with the rhCEA protein *in* vivo. Thus, a rhesus monkey CEA protein that is recombinantly expressed in a prokaryotic or eukaryotic cell and substantially purified from this host cell which does not naturally (*i.e.*, without intervention) express this rhCEA protein is of course "isolated rhesus monkey CEA protein" under any circumstances referred to herein. As noted above, a rhCEA protein preparation that is an isolated or purified rhCEA protein will be substantially free from other proteins and will contain, as a percent of its total protein, no more than 10%, preferably no more than 5%, more preferably no more than 1%, and even more preferably no more than 0.1 %, of non-rhesus monkey CEA proteins.

A "conservative amino acid substitution" refers to the replacement of one amino acid residue by another, chemically similar, amino acid residue. Examples of such conservative substitutions are: substitution of one hydrophobic residue (isoleucine, leucine, valine, or methionine) for another; substitution of one polar residue for another polar residue of the same charge (*e.g.*, arginine for lysine; glutamic acid for aspartic acid).

"rhCEA" refers to a rhesus monkey carcinoembryonic antigen.

The term "mammalian" refers to any mammal, including a human being.

The abbreviation "Ag" refers to an antigen.

The abbreviations "Ab" and "mAb" refer to an antibody and a monoclonal antibody, respectively.

The abbreviation "ORF" refers to the open reading frame of a gene.

### BRIEF DESCRIPTION OF THE DRAWINGS

FIGURE 1 shows nucleotide sequences of the rhesus monkey CEA cDNA molecules, as set forth in SEQ ID NO:1 (Panel A) and SEQ ID NO:5 (Panel B). *See* EXAMPLE 2.
FIGURE 2 shows the predicted amino acid sequences of the first rhesus monkey CEA protein, as set forth in SEQ ID NO:2 (Panel A) and the second rhesus monkey CEA protein, as set forth in SEQ ID NO:8 (Panel B). The two amino acid differences between the first and the second rhesus CEA proteins are bold and underlined in Panel B.
FIGURE 3 shows an alignment of the 5' untranslated region of human CEACAM family members. Sequences shown were compared and used to design degenerate primers as described in EXAMPLE 2. Nucleotides that are the same as the corresponding nucleotide in other CEACAM family members are highlighted. Dashes indicate that spaces were added to facilitate alignment of the sequences. Nucleotide number of each cDNA sequence, as disclosed in GenBank, is shown in parentheses.
FIGURE 4 shows the expression of the rhesus CEA protein. HeLa cells were transfected with phagemids obtained by screening the lambda-CEA library and a western blot was performed using a rabbit polyclonal antibody vs. human CEA protein. Expression of 2 clones out of 15 is shown.
FIGURE 5 shows a schematic representation of the rhesus CEA coding region. Internal repetitions are indicated and restriction sites for gene fragmentation and sequence are reported.
FIGURE 6 shows an alignment of the human (SEQ ID NO:6) and rhesus (SEQ ID NO:1) CEACAM-5 nucleotide sequences. Nucleotides that are different between the two CEACAM-5 sequences are shown in bold.
FIGURE 7 shows an alignment of the human (SEQ ID NO:7) and rhesus (SEQ ID NO:2) CEACAM-5 open reading frames. Amino acids that are different between the two CEACAM-5 sequences are shown in bold.
FIGURE 8 shows the humoral response against human CEA in CEA transgenic mice. The average antibody titer is given for two groups of mice: one immunized with rhesus CEA and one immunized with human CEA (EXAMPLE 7).
FIGURE 9 shows the cell mediated immune response against human CEA in CEA transgenic mice. CEA transgenic mice were vaccinated either with hCEA expressing vectors or with rhCEA expressing vectors (EXAMPLE 9).
FIGURE 10 shows the cell mediated immune response against rhesus CEA peptides in CEA transgenic mice immunized with rhesus or human CEA.

### DETAILED DESCRIPTION OF THE INVENTION

The gene encoding the carcinoembryonic antigen (CEA) is commonly associated with the development of adenocarcinomas. The present invention relates to compositions and methods to elicit or enhance immunity to the protein product expressed by the CEA tumor-associated antigen, wherein aberrant CEA expression is associated with the carcinoma or its development. Association of aberrant CEA expression with a carcinoma does not require that the CEA protein be expressed in tumor tissue at all timepoints of its development, as abnormal CEA expression may be present at tumor initiation and not be detectable late into tumor progression or vice-versa.

To this end, polynucleotides encoding rhesus monkey carcinoembryonic antigen (rhCEA) are provided. The molecules of the present invention may be used in a recombinant adenovirus or plasmid-based vaccine to provide effective immunoprophylaxis against adenocarcinomas through cell-mediated immunity. When directly introduced into a vertebrate *in vivo,* the invention polynucleotides induce the expression of encoded proteins within the animal, including mammals such as primates, dogs and humans.

The present invention relates to an isolated nucleic acid molecule (polynucleotide) comprising a sequence of nucleotides which encodes mRNA that expresses a novel rhCEA protein as set forth in SEQ ID NO:2 or SEQ ID NO:8. The nucleic acid molecules of the present invention are substantially free from other nucleic acids.

The isolated nucleic acid molecules of the present invention may include a deoxyribonucleic acid molecule (DNA), such as genomic DNA and complementary DNA (cDNA), which may be single (coding or noncoding strand) or double stranded, as well as synthetic DNA, such as a synthesized, single stranded polynucleotide. The isolated nucleic acid molecules of the present invention may also include a ribonucleic acid molecule (RNA). For most cloning purposes, DNA is a preferred nucleic acid.

A preferred DNA molecule of the present invention comprises the nucleotide sequence disclosed herein as SEQ ID NO:1, shown in FIGURE 1A, which encodes the rhesus CEA protein shown in FIGURE 2A and set forth as SEQ ID NO:2.

Another preferred DNA molecule of the present invention comprises the nucleotide sequence disclosed herein as SEQ ID NO:5 (hereinafter "second rhCEA" DNA sequence), shown in FIGURE 1B, which encodes the rhesus CEA protein shown in FIGURE 2B and set forth as SEQ ID NO:8. These rhCEA nucleic acid molecules were identified through RT-PCR as described in detail in EXAMPLE 2. The second rhCEA DNA sequence (SEQ ID NO:5) differs from the first by two nucleotides and was cloned from colon tissue from a different rhesus monkey. This DNA sequence codes for a rhesus CEA protein that differs from the first rhesus CEA protein by two amino acids.

The isolated cDNA clones, associated vectors, host cells recombinant subcellular fractions and membranes, and the expressed and mature forms of rhCEA are useful for the development of a cancer vaccine.

The present invention also includes biologically active fragments or mutants of SEQ ID NOs:1 or 5, which encode mRNA expressing novel rhCEA proteins. Any such biologically active fragment and/or mutant will encode either a protein or protein fragment which at least substantially mimics the pharmacological properties of the rhCEA protein, including but not limited to the rhCEA protein as set forth in SEQ ID NO:2 or SEQ ID NO:8. Any such polynucleotide includes but is not necessarily limited to: nucleotide substitutions, deletions, additions, amino-terminal truncations and carboxy-terminal truncations. The mutations of the present invention encode mRNA molecules that express a functional rhCEA protein in a eukaryotic cell so as to be useful in cancer vaccine development.

This invention also relates to synthetic DNA that encodes the rhCEA protein where the nucleotide sequence of the synthetic DNA differs significantly from the nucleotide sequence of SEQ ID NO:1 and SEQ ID NO:5, but still encodes the rhCEA protein as set forth in SEQ ID NO:2 or SEQ ID NO:8. Such synthetic DNAs are intended to be within the scope of the present invention.

Therefore, the present invention discloses codon redundancy that may result in numerous DNA molecules expressing an identical protein. For purposes of this specification, a sequence bearing one or more replaced codons will be defined as a degenerate variation. Also included within the scope of this invention are mutations either in the DNA sequence or the translated protein that do not substantially alter the ultimate physical properties of the expressed protein. For example, substitution of valine for leucine, arginine for lysine, or asparagine for glutamine may not cause a change in the functionality of the polypeptide.

It is known that DNA sequences coding for a peptide may be altered so as to code for a peptide that has properties that are different than those of the naturally occurring peptide. Methods of altering the DNA sequences include but are not limited to site directed mutagenesis. Examples of altered properties include but are not limited to changes in the affinity of an enzyme for a substrate or receptor for a ligand.

Included in the present invention are DNA sequences that hybridize to SEQ ID NO:1 or SEQ ID NO:5 under stringent conditions. By way of example, and not limitation, a procedure using conditions of high stringency is as follows: Prehybridization of filters containing DNA is carried out for 2 hours to overnight at 65°C in buffer composed of 6X SSC, 5X Denhardt's solution, and 100 µg/ml denatured salmon sperm DNA. Filters are hybridized for 12 to 48 hrs at 65°C in prehybridization mixture containing 100 µg/ml denatured salmon sperm DNA and 5-20 X 10⁶ cpm of ³²P-labeled probe. Washing of filters is done at 37°C for 1 hr in a solution containing 2X SSC, 0.1% SDS. This is followed by a wash in 0.1X SSC, 0.1% SDS at 50°C for 45 min. before autoradiography. Other procedures using conditions of high stringency would include either a hybridization step carried out in 5XSSC, 5X Denhardt's solution, 50% formamide at 42°C for 12 to 48 hours or a washing step carried out in 0.2X SSPE, 0.2% SDS at 65°C for 30 to 60 minutes.

Reagents mentioned in the foregoing procedures for carrying out high stringency hybridization are well known in the art. Details of the composition of these reagents can be found in, e.g., Sambrook et al., *Molecular Cloning: A Laboratory Manual;* Cold Spring Harbor Laboratory, Cold Spring Harbor, New York, 1989, which is hereby incorporated by reference. In addition to the foregoing, other conditions of high stringency which may be used are well known in the art.

A preferred aspect of the present invention is a substantially purified form of a rhesus monkey CEA protein which comprises a sequence of amino acids as disclosed in FIGURE 2A (SEQ ID NO:2).

Another preferred aspect of the present invention is a substantially purified form of a rhesus monkey CEA protein which comprises a sequence of amino acids as disclosed in FIGURE 2B (SEQ ID NO:8).

This invention also relates to various functional domains of rhCEA and to hybrid molecules comprising at least one of these sequences. The CEA protein comprises an amino-terminal domain with a processed leader sequence and a hydrophobic carboxy-terminal domain. CEA also comprises three Ig-like internal domains. Subdomains of the N-terminal domain were shown by Taheri et al. (*J. Biol. Chem.* 275(35): 26935-26943 (2000)) to be required for CEA's intercellular adhesion function.

The present invention also includes biologically active fragments and/or mutants of a rhCEA protein, comprising the amino acid sequence as set forth in SEQ ID NO: 2 or SEQ ID NO:8, including but not necessarily limited to amino acid substitutions, deletions, additions, amino terminal truncations and carboxy-terminal truncations such that these mutations provide for proteins or protein fragments of diagnostic, therapeutic or prophylactic use and would be useful for cancer vaccine development.

The rhesus monkey CEA proteins of the present invention may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

The present invention also relates to rhCEA fusion constructs, including but not limited to fusion constructs which express a portion of the rhesus CEA protein linked to various markers, including but in no way limited to GFP (Green fluorescent protein), the MYC epitope, GST, and Fc. Any such fusion construct may be expressed in the cell line of interest and used to screen for modulators of the rhesus CEA protein disclosed herein. Also contemplated are fusion constructs that are constructed to enhance the immune response to rhesus CEA including, but not limited to: DOM and hsp70.

The present invention further relates to recombinant vectors that comprise the substantially purified nucleic acid molecules disclosed throughout this specification. These vectors may be comprised of DNA or RNA. For most cloning purposes, DNA vectors are preferred. Typical vectors include plasmids, modified viruses, bacteriophage, cosmids, yeast artificial chromosomes, and other forms of episomal or integrated DNA that can encode a rhCEA protein. It is well within the purview of the skilled artisan to determine an appropriate vector for a particular gene transfer or other use.

An expression vector containing DNA encoding a rhCEA protein may be used for expression of rhCEA in a recombinant host cell. Expression vectors may include, but are not limited to, cloning vectors, modified cloning vectors, specifically designed plasmids or viruses. Also, a variety of bacterial expression vectors may be used to express recombinant rhCEA in bacterial cells if desired. In addition, a variety of fungal cell expression vectors may be used to express recombinant rhCEA in fungal cells. Further, a variety of insect cell expression vectors may be used to express recombinant protein in insect cells.

The present invention also relates to host cells transformed or transfected with vectors comprising the nucleic acid molecules of the present invention. Recombinant host cells may be prokaryotic or eukaryotic, including but not limited to, bacteria such as *E. coli,* fungal cells such as yeast, mammalian cells including, but not limited to, cell lines of bovine, porcine, monkey and rodent origin; and insect cells including but not limited to *Drosophila* and silkworm derived cell lines. Such recombinant host cells can be cultured under suitable conditions to produce rhCEA or a biologically equivalent form.

As noted above, an expression vector containing DNA encoding a rhCEA protein may be used for expression of rhCEA in a recombinant host cell. Therefore, another aspect of this invention is a process for expressing a rhesus monkey CEA protein in a recombinant host cell, comprising: (a) introducing a vector comprising a nucleic acid as set forth in SEQ ID NO:1 or SEQ ID NO:5 into a suitable host cell; and, (b) culturing the host cell under conditions which allow expression of said rhesus monkey CEA protein.

Following expression of rhCEA in a host cell, rhCEA protein may be recovered to provide rhCEA protein in active form. Several rhCEA protein purification procedures are available and suitable for use. Recombinant rhCEA protein may be purified from cell lysates and extracts by various combinations of, or individual application of salt fractionation, ion exchange chromatography, size exclusion chromatography, hydroxylapatite adsorption chromatography and hydrophobic interaction chromatography. In addition, recombinant rhCEA protein can be separated from other cellular proteins by use of an immunoaffinity column made with monoclonal or polyclonal antibodies specific for full-length rhCEA protein, or polypeptide fragments of rhCEA protein.

The nucleic acids of the present invention may be assembled into an expression cassette which comprises sequences designed to provide for efficient expression of the protein in a human cell. The cassette preferably contains the full-length rhCEA gene, with related transcriptional and translations control sequences operatively linked to it, such as a promoter, and termination sequences. In a preferred embodiment, the promoter is the cytomegalovirus promoter without the intron A sequence (CMV), although those skilled in the art will recognize that any of a number of other known promoters such as the strong immunoglobulin, or other eukaryotic gene promoters may be used. A preferred transcriptional terminator is the bovine growth hormone terminator, although other known transcriptional terminators may also be used. The combination of CMV-BGH terminator is particularly preferred.

In accordance with this invention, the rhesus CEA expression cassette is inserted into a vector. The vector is preferably an adenoviral vector, although linear DNA linked to a promoter, or other vectors, such as adeno-associated virus or a modified vaccinia virus, retroviral or lentiviral vector may also be used.

If the vector chosen is an adenovirus, it is preferred that the vector be a so-called first-generation adenoviral vector. These adenoviral vectors are characterized by having a non-functional E1 gene region, and preferably a deleted adenoviral E1 gene region. In some embodiments, the expression cassette is inserted in the position where the adenoviral E1 gene is normally located. In addition, these vectors optionally have a non-functional or deleted E3 region. It is preferred that the adenovirus genome used be deleted of both the E1 and E3 regions (ΔE1ΔE3). The adenoviruses can be multiplied in known cell lines which express the viral E1 gene, such as 293 cells, or PERC.6 cells, or in cell lines derived from 293 or PERC.6 cell which are transiently or stablily transformed to express an extra protein. For examples, when using constructs that have a controlled gene expression, such as a tetracycline regulatable promoter system, the cell line may express components involved in the regulatory system. One example of such a cell line is T-Rex-293; others are known in the art.

For convenience in manipulating the adenoviral vector, the adenovirus may be in a shuttle plasmid form. This invention is also directed to a shuttle plasmid vector which comprises a plasmid portion and an adenovirus portion, the adenovirus portion comprising an adenoviral genome which has a deleted E1 and optional E3 deletion, and has an inserted expression cassette comprising rhesus CEA. In preferred embodiments, there is a restriction site flanking the adenoviral portion of the plasmid so that the adenoviral vector can easily be removed. The shuttle plasmid may be replicated in prokaryotic cells or eukaryotic cells.

In a preferred embodiment of the invention, the expression cassette is inserted into the pMRKAd5-HV0 adenovirus plasmid (See Emini et al., WO 02/22080, which is hereby incorporated by reference). This plasmid comprises an Ad5 adenoviral genome deleted of the E1 and E3 regions. The design of the pMRKAd5-HVO plasmid was improved over prior adenovectors by extending the 5' cis-acting packaging region further into the E1 gene to incorporate elements found to be important in optimizing viral packaging, resulting in enhanced virus amplification. Advantageously, this enhanced adenoviral vector is capable of maintaining genetic stability following high passage propagation.

Standard techniques of molecular biology for preparing and purifying DNA constructs enable the preparation of the adenoviruses, shuttle plasmids, and DNA immunogens of this invention.

The vectors described above may be used in immunogenic compositions and vaccines for preventing the development of adenocarcinomas associated with aberrant CEA expression and/or for treating existing cancers. To this end, one aspect of the instant invention is a method of preventing or treating cancer comprising administering to a mammal a vaccine vector comprising an isolated nucleic acid molecule, the isolated nucleic acid molecule comprising a sequence of nucleotides that encodes a rhesus monkey CEA protein as set forth in SEQ ID NO:2 or SEQ ID NO:8.

In accordance with the method described above, the vaccine vector may be administered for the treatment or prevention of cancer in any mammal. In a preferred embodiment of the invention, the mammal is a human.

Further, one of skill in the art may choose any type of vector for use in the treatment and prevention method described. Preferably, the vector is an adenovirus vector or a plasmid vector. In a preferred embodiment of the invention, the vector is an adenoviral vector comprising an adenoviral genome with a deletion in the adenovirus E1 region, and an insert in the adenovirus E1 region, wherein the insert comprises an expression cassette comprising: (a) a polynucleotide encoding a rhesus monkey CEA protein; and (b) a promoter operably linked to the polynucleotide.

The instant invention further relates to an adenovirus vaccine vector comprising an adenoviral genome with a deletion in the E1 region, and an insert in the E1 region, wherein the insert comprises an expression cassette comprising: (a) a polynucleotide encoding a rhesus monkey CEA protein; and (b) a promoter operably linked to the polynucleotide.

In a preferred embodiment of this aspect of the invention, the adenovirus vector is an Ad 5 vector.

In another preferred embodiment of the invention, the adenovirus vector is an Ad 6 vector.

In another aspect, the invention relates to a vaccine plasmid comprising a plasmid portion and an expression cassette portion, the expression cassette portion comprising: (a) a polynucleotide encoding a rhesus monkey CEA protein; and (b) a promoter operably linked to the polynucleotide.

In some embodiments of this invention, the recombinant adenovirus vaccines disclosed herein are used in various prime/boost combinations with a plasmid-based polynucleotide vaccine in order to induce an enhanced immune response. In this case, the two vectors are administered in a "prime and boost" regimen. For example the first type of vector is administered, then after a predetermined amount of time, for example, 1 month, 2 months, six months, or other appropriate interval, a second type of vector is administered. Preferably the vectors carry expression cassettes encoding the same polynucleotide or combination of polynucleotides. In the embodiment where a plasmid DNA is also used, it is preferred that the vector contain one or more promoters recognized by mammalian or insect cells. In a preferred embodiment, the plasmid would contain a strong promoter such as, but not limited to, the CMV promoter. The rhesus CEA gene or other gene to be expressed would be linked to such a promoter. An example of such a plasmid would be the mammalian expression plasmid V lJns as described (J. Shiver *et. al.* in *DNA Vaccines,* M. Liu et al. eds., N.Y. Acad. Sci., N.Y., 772:198-208 (1996), which is herein incorporated by reference).

As stated above, an adenoviral vector vaccine and a plasmid vaccine may be administered to a vertebrate as part of a single therapeutic regime to induce an immune response. To this end, the present invention relates to a method of protecting a mammal from cancer comprising: (a) introducing into the mammal a first vector comprising: i) a polynucleotide encoding a rhesus monkey CEA protein; and ii) a promoter operably linked to the polynucleotide; (b) allowing a predetermined amount of time to pass; and (c) introducing into the mammal a second vector comprising: i) a polynucleotide encoding a rhesus monkey CEA protein; and ii) a promoter operably linked to the polynucleotide.

In one embodiment of the method of protection described above, the first vector is a plasmid and the second vector is an adenovirus vector. In an alternative embodiment, the first vector is an adenovirus vector and the second vector is a plasmid.

The instant invention further relates to a method of treating a mammal suffering from an adenocarcinoma comprising: (a) introducing into the mammal a first vector comprising: i) a polynucleotide encoding a rhesus monkey CEA protein; and ii) a promoter operably linked to the polynucleotide; (b) allowing a predetermined amount of time to pass; and (c) introducing into the mammal a second vector comprising: i) a polynucleotide encoding a rhesus monkey CEA protein; and ii) a promoter operably linked to the polynucleotide.

In one embodiment of the method of treatment described above, the first vector is a plasmid and the second vector is an adenovirus vector. In an alternative embodiment, the first vector is an adenovirus vector and the second vector is a plasmid.

The amount of expressible DNA or transcribed RNA to be introduced into a vaccine recipient will depend partially on the strength of the promoters used and on the immunogenicity of the expressed gene product. In general, an immunologically or prophylactically effective dose of about 1 ng to 100 mg, and preferably about 10 µg to 300 µg of a plasmid vaccine vector is administered directly into muscle tissue. An effective dose for recombinant adenovirus is approximately 106 -101² particles and preferably about 10⁷―10¹¹particles. Subcutaneous injection, intradermal introduction, impression though the skin, and other modes of administration such as intraperitoneal, intravenous, or inhalation delivery are also contemplated. It is also contemplated that booster vaccinations may be provided. Parenteral administration, such as intravenous, intramuscular, subcutaneous or other means of administration with adjuvants such as interleukin 12 protein, concurrently with or subsequent to parenteral introduction of the vaccine of this invention is also advantageous.

The vaccine vectors of this invention may be naked, i.e., unassociated with any proteins, adjuvants or other agents which impact on the recipient's immune system. In this case, it is desirable for the vaccine vectors to be in a physiologically acceptable solution, such as, but not limited to, sterile saline or sterile buffered saline Alternatively, it may be advantageous to administer an immunostimulant, such as an adjuvant, cytokine, protein, or other carrier with the vaccines or immunogenic compositions of the present invention. Therefore, this invention includes the use of such immunostimulants in conjunction with the compositions and methods of the present invention. An immunostimulant, as used herein, refers to essentially any substance that enhances or potentiates an immune response (antibody and/or cell-mediated) to an exogenous antigen. Said immunostimulants can be administered in the form of DNA or protein. Any of a variety of immunostimulants may be employed in conjunction with the vaccines and immunogenic compositions of the present inventions, including, but not limited to: GM-CSF, IFNα, tetanus toxoid, IL12, B7.1, LFA-3 and ICAM-1. Said immunostimulants are well-known in the art. Agents which assist in the cellular uptake of DNA, such as, but not limited to calcium ion, may also be used. These agents are generally referred to as transfection facilitating reagents and pharmaceutically acceptable carriers. Those of skill in the art will be able to determine the particular immunostimulant or pharmaceutically acceptable carrier as well as the appropriate time and mode of administration.

Any of a variety of procedures may be used to clone rhCEA. These methods include, but are not limited to, (1) a RACE PCR cloning technique (Frohman et al., *Proc. Natl. Acad. Sci. USA* 85: 8998-9002 (1988)). 5' and/or 3' RACE may be performed to generate a full-length cDNA sequence. This strategy involves using gene-specific oligonucleotide primers for PCR amplification of rhCEA cDNA. These gene-specific primers are designed through identification of an expressed sequence tag (EST) nucleotide sequence which has been identified by searching any number of publicly available nucleic acid and protein databases; (2) direct functional expression of the rhCEA cDNA following the construction of a rhCEA-containing cDNA library in an appropriate expression vector system; (3) screening an rhCEA-containing cDNA library constructed in a bacteriophage or plasmid shuttle vector with a labeled degenerate oligonucleotide probe designed from the amino acid sequence of the rhCEA protein; (4) screening an rhCEA-containing cDNA library constructed in a bacteriophage or plasmid shuttle vector with a partial cDNA encoding the rhCEA protein. This partial cDNA is obtained by the specific PCR amplification of rhCEA DNA fragments through the design of degenerate oligonucleotide primers from the amino acid sequence known for other membrane proteins which are related to the rhCEA protein; (5) screening a rhCEA-containing cDNA library constructed in a bacteriophage or plasmid shuttle vector with a partial cDNA or oligonucleotide with homology to a mammalian rhCEA protein. This strategy may also involve using gene-specific oligonucleotide primers for PCR amplification of rhCEA cDNA identified as an EST as described above; or (6) designing 5' and 3' gene specific oligonucleotides using SEQ ID NO: 1 as a template so that either the full-length cDNA may be generated by known RACE techniques, or a portion of the coding region may be generated by these same known RACE techniques to generate and isolate a portion of the coding region to use as a probe to screen one of numerous types of cDNA and/or genomic libraries in order to isolate a full-length version of the nucleotide sequence encoding rhCEA.

It is readily apparent to those skilled in the art that other types of libraries, as well as libraries constructed from other cell types-or species types, may be useful for isolating a rhCEA-encoding DNA or a rhCEA homologue. Other types of libraries include, but are not limited to, cDNA libraries derived from other cells. The selection of cells or cell lines for use in preparing a cDNA library to isolate a cDNA encoding rhCEA may be done by first measuring cell-associated rhCEA activity using any known assay available for such a purpose.

Preparation of cDNA libraries can be performed by standard techniques well known in the art. Well known cDNA library construction techniques can be found for example, in Sambrook et al., *Molecular Cloning: A Laboratory Manual;* Cold Spring Harbor Laboratory, Cold Spring Harbor, New York, 1989. Complementary DNA libraries may also be obtained from numerous commercial sources, including but not limited to Clontech Laboratories, Inc. (Palo Alto, CA) and Stratagene (La Jolla, CA).

The DNA molecules, RNA molecules, and recombinant protein of the present invention may be used to screen and measure levels of rhCEA. The recombinant proteins, DNA molecules, and RNA molecules lend themselves to the formulation of kits suitable for the detection and typing of rhCEA. Such a kit would comprise a compartmentalized carrier suitable to hold in close confinement at least one container. The carrier would further comprise reagents such as recombinant rhCEA or anti-rhCEA antibodies suitable for detecting rhCEA. The carrier may also contain a means for detection such as labeled antigen or enzyme substrates or the like.

Having described preferred embodiments of the invention with reference to the accompanying drawings, it is to be understood that the invention is not limited to those precise embodiments, and that various changes and modifications may be effected therein by one skilled in the art without departing from the scope or spirit of the invention as defined in the appended claims.

### EXAMPLE 1

### Isolation of RNA from Rhesus Macaques

Molecular procedures were performed following standard procedures well known in the art (*See, e.g.,* Ausubel et. al. *Short Protocols in Molecular Biology,* F.M., -2^{nd}. ed., John Wiley & Sons, (1992) and Sambrook et al., *Molecular Cloning, A Laboratory Manual,* 2^{nd} ed., Cold Spring Harbor Laboratory Press (1989), which are hereby incorporated by reference).

To obtain RNA for the isolation of the rhesus CEA cDNA, colon samples from two different Rhesus monkeys *(Macaca Mulatta)* were used. Frozen tissues were obtained from The Biomedical Primate Research Center (BPRC, Rijswijk, the Netherlands). To extract total RNA from rhesus colon samples, tissues were mechanically pulverized and combined with the Ultraspec RNA reagent (Biotecx Laboratories; Houston, TX) according to the manufacturer's instructions. The integrity of the purified RNA was verified by formaldehyde-denaturing agarose gel. Samples were aliquoted and stored at -80°C.

### EXAMPLE 2

### Rhesus CEA cDNA Amplification

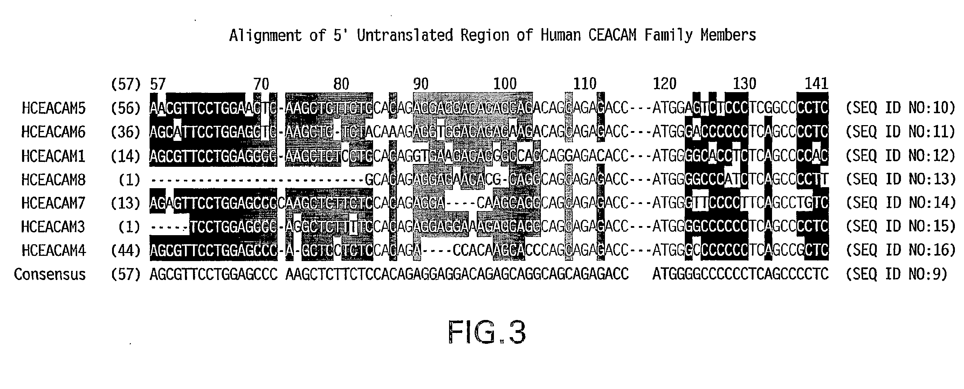

Nucleotide sequences from the 5' and 3' untranslated regions (UTR) of all known members of the human CEA family were aligned to identify highly conserved regions of the CEA DNA (see FIGURE 3). Based on the CEA gene family homologies identified, degenerate oligonucleotide primers were designed and PCR conditions were optimized to amplify the rhesus CEA cDNA by reverse transcriptase polymerase chain reaction (RT-PCR), described below. The primers used to amplify the entire cDNA were as follows: 5'-RhCEA EcoRI 5'-CCGAATTCCGGAC ASAGCAGRCAGCAGRSACC-3'(SEQ ID NO:3) and CEA-8 RhXhoI 5'-CCGCTCGAGCGGCTGCTACATCAGAGCAACCCCAA C C -3'(SEQ ID NO:4). The amplification was performed with the SuperScript One-Step RT-PCR with Platinum Taq kit (Invitrogen; Carlsbad, CA). A 100 µl reaction volume was used which consisted of 1 µg of RNA, 200pmol of both primers, and 10% DMSO (final concentration).

To perform the reverse transcription step, total RNA samples isolated from each of the two rhesus monkeys were incubated at 45°C for 30 min, followed by a 2 minute incubation at 94°C. PCR amplification of the resulting templates consisted of 40 cycles of 94°C for 15s, 52°C for 30s and 68°C for 2 min and 20s.

Amplified PCR products of about 2100 bp, the expected size for a CEACAM-5 homolog, were independently obtained from both RNA samples and were purifed from agarose gel. Partial sequence analysis of both PCR products revealed high homology with human CEACAM-5.

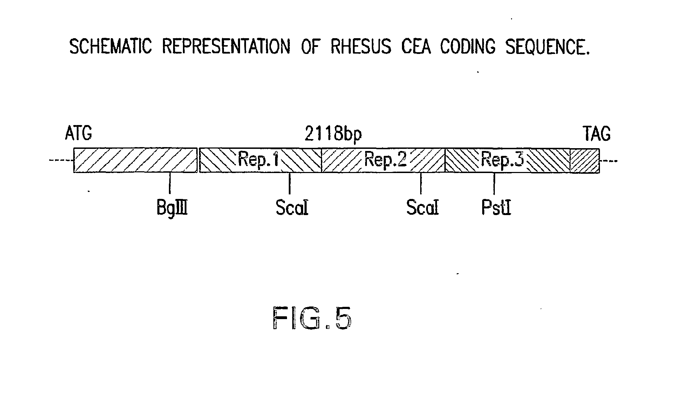

Due to the high homology of internal repetitions, the entire gene sequence was obtained by purifying DNA fragments using the restriction sites indicated in FIGURE 5. The rhesus CEA nucleotide sequences obtained from each monkey are disclosed herein in FIGURE 1, as set forth in SEQ ID NO:1 (hereinafter rhCEACAM-5) and SEQ ID NO:5 (hereinafter rhCEACAM-5 #2). Analysis of the CEA nucleotide sequences revealed an open reading frame (ORF) of 2118 nucleotides, which encode a 705 amino acid polypeptide. Comparison of the rhCEA nucleotide sequences obtained from two rhesus monkeys indicated that there were two nucleotide differences (see FIGURES 1A and 1B), which code for two different proteins (see FIGURES 2A and 2B).

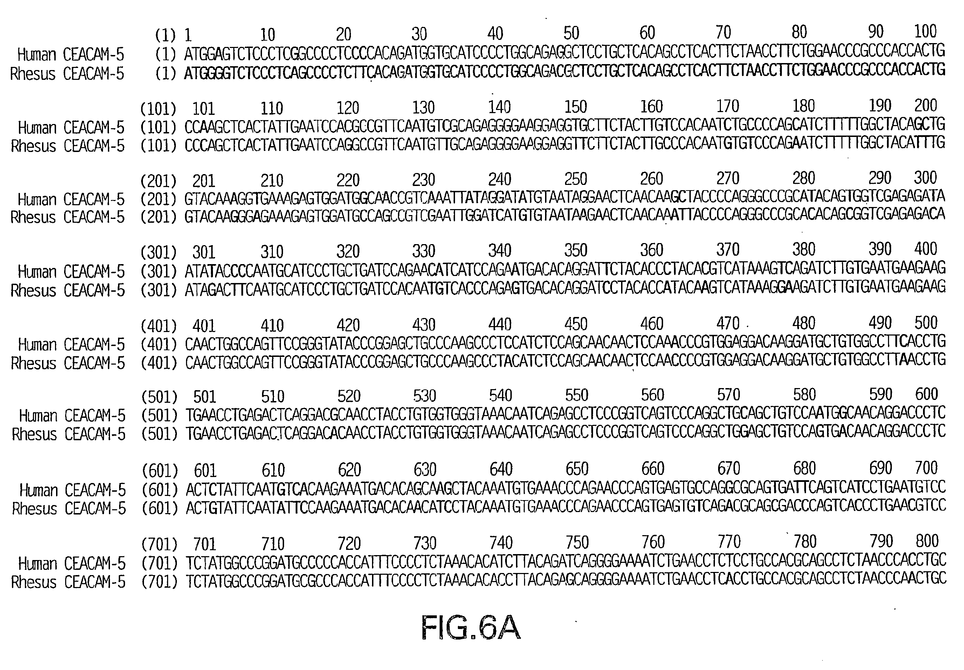

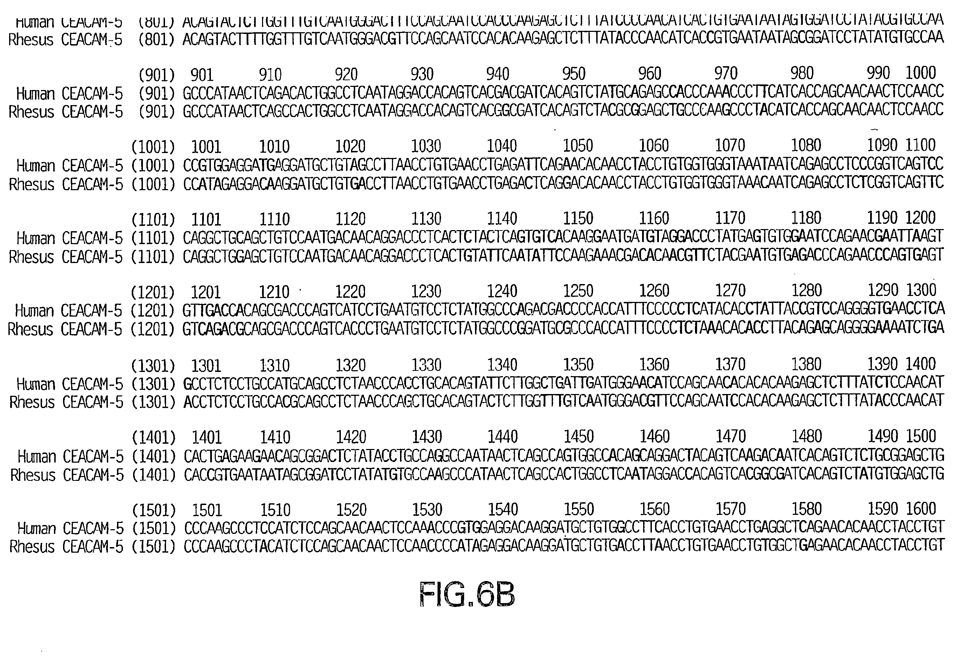

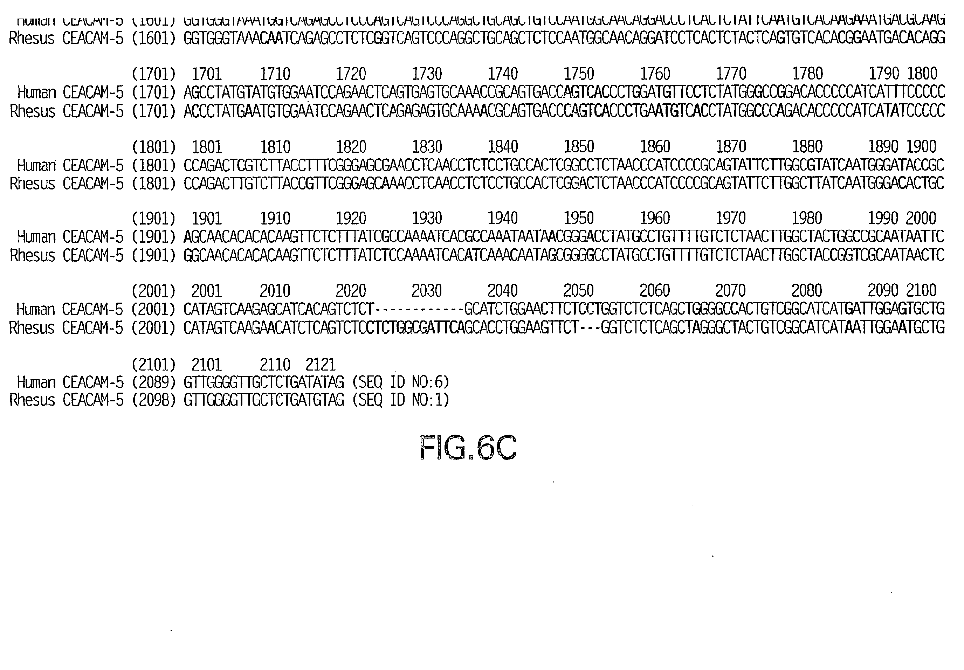

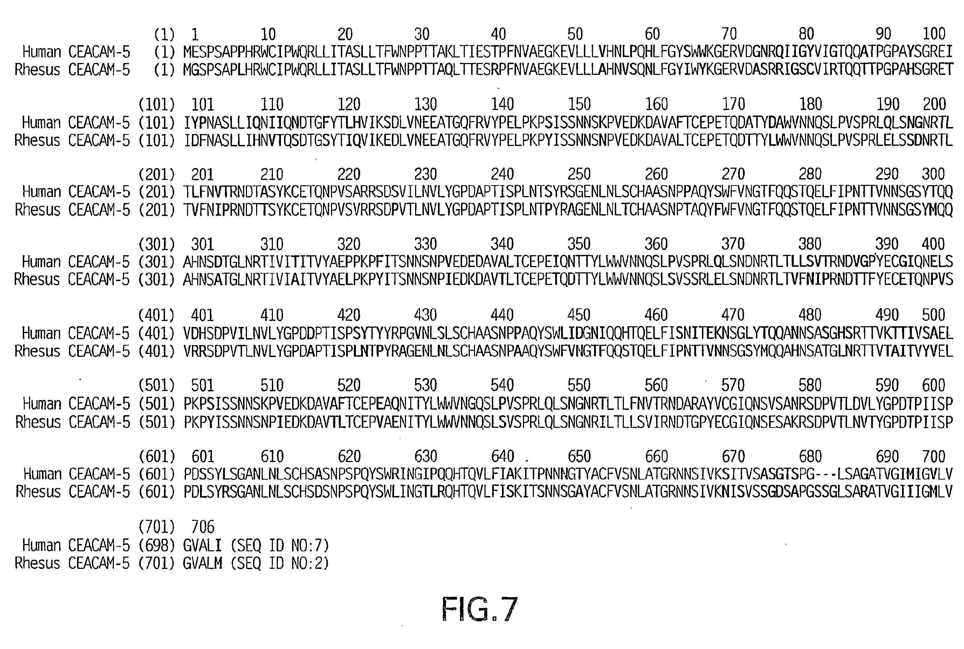

The rhesus CEACAM-5 nucleotide sequence (SEQ ID NO:1) was also compared to the published human CEACAM-5 sequence (SEQ ID NO:6), which revealed 88% homology at the nucleotide level (see FIGURE 6). A similar comparison of the rhesus (SEQ ID NO:2) and human (SEQ ID NO:7) CEA polypeptide sequences showed 78.9% identity at the amino acid level (see FIGURE 7). Interestingly, a three amino acid insertion is present in the carboxyl-terminus of rhesus CEA compared to human CEA, probably involving the signal for glycosylphosphatidylinosital (GPI) modification.

### EXAMPLES 3

### Generation and Screening of a Lambda Rhesus CEA-Specific Library.

Amplified rhCEA products obtained by RT-PCR (see EXAMPLE 2) were digested with *Eco*RI/*Xho*I and ligated into the Lambda ZAP-CMV XR vector (Stratagene; La Jolla, CA), according to manufacturer's directions. The ligation products were incubated with Gigapack III gold packaging extract and the resulting phages were used to infect XL-1 Blue MRF' cells. This CEA-specific primary library was then amplified, obtaining a titer of ~1x10⁶ pfu/ml. Screening of ~5x10³ plaques was performed by lifting onto nylon filters. Filters were hybridized with two different DNA probes covering the 5' and the 3' ends of the CEA molecule. Double positive plaques were excised in XL-1 Blue MRF' cells and the derived filamentous phages were amplified in XL-OLR cells. The phagemids were then grown and analyzed by restriction digestion. Sequence analysis and Genbank comparisons revealed the highest homology with human CEACAM-5.

### EXAMPLE 4

### Plasmid Constructs and Adenovirus Generation

RhCEA was excised with *Pst*I/*Xho*I from pCMV-script EX phagemid vector and inserted in pBluescript II KS vector, obtaining pBS-RhCEA. The insert was entirely sequenced and then subcloned as *Sma*I/*Xho*I fragment in pVIJnsA vector, obtaining pVIJ-RhCEA. The shuttle plasmid pNIRK-RhCEA for adenovirus generation was obtained by subcloning the same fragment in the polyMRK vector. A *Pac*I/*Stu*I fragment from pMRK-RhCEA containing the expression cassette for RhCEA and E1 flanking Ad5 regions was recombined to *Cla*I linearized pAd5 or pAd6 in BJ5183 *E. Coli* cells. The resulting plasmids were pAd5-RhCEA and pAd6-RhCEA. Both plasmids were cut with *Pac*I to release the adenovirus ITRs and transfected in PerC-6 cells. Viral amplification was carried out through serial passages. Ad5-RhCEA and Ad6-RhCEA were purified using a standard CsCl purification protocol and extensively dyalized against A105 buffer (5mM Tris pH 8.0, 1mM MgCl2, 75mM NaCl, 5% sucrose, 0.005% Tween20).

### EXAMPLE 5

### RhCEA Expression and Detection in vitro

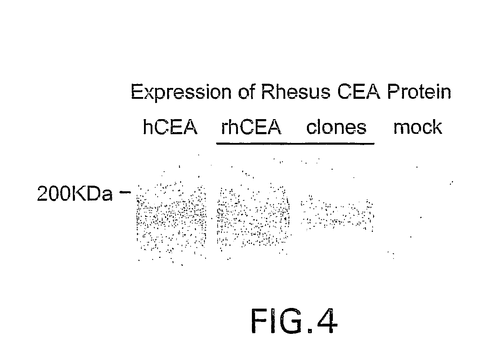

Expression of RhCEA by the generated vectors was verified by western blot and FACS analysis. Plasmids were transfected in HeLa or PerC.6 cells with Lipofectamine 2000 (Life Technologies; Carlsbad, CA). Adenovirus infections were performed in serum-free medium for 30 min at 37°C, then fresh medium was added. After 48hr of incubation, whole cell lysates were analyzed by western blot using a rabbit polyclonal serum against human CEA (Fitzgerald, 1:1500 dilution). All of the selected rhesus CEA clones expressed a 180-200 KDa protein when transfected in HeLa cells (see FIGURE 4).

For FACS analysis, cells were detached with trypsin and resuspended in FACS buffer (PBS, 1% FCS). After incubation for 30 min with rabbit polyclonal anti-CEA antibody diluted 1:250, cells were washed and incubated for 30 min with an anti-rabbit IgG-PE and finally analyzed with a FACScalibur (Becton Dickinson, San Jose, CA).

### EXAMPLE 6

### Peptides

In order to analyze the cell mediated immune response against rhesus CEA in immunized animals, 15mer peptides overlapping by 11 amino acids were designed to cover the entire protein. Liophylized rhesus CEA peptides were purchased by Bio-Synthesis, Inc. (Lewisville, TX) and resuspended in DMSO at 40mg/ml. Peptides were grouped into 4 pools: pool A (from RhCEA-1 to RhCEA-34, 34 peptides); pool B (from RhCEA-35 to RhCEA-79, 45 peptides); pool C (from RhCEA-80 to RhCEA-124, 48 peptides); and pool D (from RhCEA-125 to RhCEA-173, 53 peptides). Final concentrations were the following: pool A=1.176mg/ml; pool B=0.888mg/ml; pool C=0.851mg/ml; pool D=0.769mg/ml. Peptides and pools were stored at -80°C.

### EXAMPLE 7

### Generation of CEA-specific cellular immune responses in mice by immunization with rhCEA

CEA.Tg mice are transgenic mice that express human CEA as a self-antigen with a tissue distribution similar to that of humans. As largely demonstrated in the scientific literature, these mice are unresponsive to CEA, as shown by the lack of detectable CEA-specific serum antibodies and the inability to prime an *in vitro* splenic T-cell response to CEA. Many reports have shown that DNA immunization with xenogeneic genes encoding homologous antigens protects mice against tumor challenge with syngeneic melanoma cells. To demonstrate the capability of xenogeneic DNA vaccination to elicit an immune response against a self-antigen in this model, we immunized CEA.Tg mice with vectors encoding rhesus CEA (xeno).

C57BL/6 mice (H-2^{b}) were purchased from Charles River (Lecco, Italy). CEA.tg mice (H-2b) were provided by HL Kaufman (Albert Einstein College of Medicine, New York) and kept in standard conditions.

For electro gene transfer (EGT), mice quadriceps were either surgically exposed or directly injected with 50 µg pVIJ-RhCEA and electrically stimulated as previously described (Rizzuto at al. *Proc. Natl. Acad. Sci. U.S.A.* 96(11): 6417-22 (1999)). For adenovirus injection, 1x10¹⁰ vp of Ad5-RhCEA were injected in mice quadriceps.

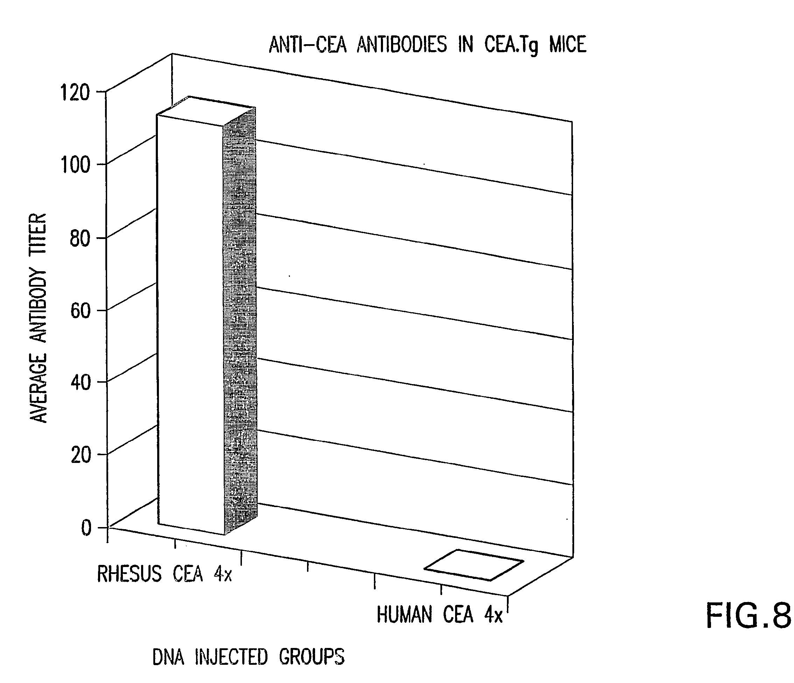

Mice were injected in the quadriceps muscle with 50µg pVIJ-RhCEA and electrostimulated immediately after injection once a week for 4 weeks. C57BL/6 mice were used as controls. Antibodies against rhesus CEA were detected in sera from these mice by western blot, demonstrating a humoral immune response. A mouse monoclonal Ab against hCEA was used as positive control, while pre-immune sera and mock-infected cell extracts were used as negative controls (data not shown). Importantly, cross-reactive antibodies against human CEA protein could be measured only in rhesus CEA immunized groups (FIGURE 8) with an average titer of 1:110. These data indicate that, in the transgenic mouse model, it is possible to break tolerance with xenogeneic DNA vaccination (measured as anti-CEA autoantibodies).

### EXAMPLE 8

### Antibody Detection and Titration

Sera for antibody titration were obtained by retro-orbital bleeding. For western blot detection, extracts from HeLa cells transduced with Ad5-rhCEA were run on SDS-page gels and transferred onto nitrocellulose filters. Sera were pooled and diluted 1:50 for O/N incubation at 4°C. An anti-mouse IgG-AP conj. (Sigma, 1:2500) was used for the detection. For titration, Elisa plates (Nunc maxisorp) were coated with 100ng/well CEA (highly pure CEA; Fitzgerald Industries International Inc., Concord MA), diluted in coating buffer (50mM NaHCO₃ pH 9.4) and incubated O/N at 4°C. Plates were then blocked with PBS containing 5% BSA for 1 hr at 37°C. Mouse sera were diluted in PBS 5% BSA (dilution 1/50 to evaluate seroconversion rate; dilutions from 1:10 to 1:31,250 to evaluate titre value). Pre-immune sera were used as background. Diluted sera were incubated O/N at 4°C. Washes were carried out with PBS, 1%BSA, 0.05% tween 20. Detecting antibody (goat anti-mouse IgG Peroxidase, Sigma, St. Louis, MO) was diluted 1/2000 in PBS, 5%BSA.) and incubated for 2-3 hr at room temp. on a shaker. After washing, plates were developed with 100µl/well of TMB substrate (Pierce Biotechnology, Inc., Rockford, IL). Reactions were stopped with 25µl/well of 1M H₂SO₄ solution and plates were read at 450 nm/620 nm. Anti-CEA serum titers were calculated as the limiting dilution of serum producing an absorbance at least 3-fold greater than the absorbance of autologous pre-immune serum at the same dilution.

### EXAMPLE 9

### EFN-γ ELISPOT Assay

96-well MAIP plates (Millipore, Bedford, MA) were coated with purified rat anti-mouse IFN-γ (IgG1, clone R4-6A2, Pharmingen, San Diego, CA) at 2.5µg/ml in sterile PBS, aliquoted at 100µl per well. After washing with sterile PBS, plates were blocked with 200µl per well of R10 medium at 37 °C for at least 2 hours.

For splenocyte preparation, the spleen was removed from a sacrificed mouse in a sterile manner and disrupted by scratching through a grid. Osmotic lysis of red blood cells was obtained by adding 1 ml of 0.1X PBS to the cell pellet and vortexing for no more than 15 sec. 1 ml of 2X PBS was then added and the volume was brought up to 4ml with PBS 1X. After spinning at 1200 rpm for 10 minutes at room temp., the cell pellet was resuspended in 1 ml of R10 medium and viable cells were counted. Splenocytes were plated at 5x10⁵ and 2x10⁵/well with 1µg/ml each peptide in R10 and incubated for 20h in a CO₂ incubator at 37°C. Concanavalin A (ConA) at 5µg/ml was used as a positive internal control for each mouse. After washing with PBS, 0.05% Tween 20, plates were incubated O/N at 4°C with 50µl/well of biotin-conjugated rat anti-mouse IFN-γ (Rat IgG1, clone XMG 1.2, Pharmingen, San Jose, CA) diluted 1:250 in assay buffer (PBS-5%FBS - 0.005% Tween-20).

The next day, plates were washed and incubated for 2h at room temp. with Streptavidin-AP conjugate (Pharmingen) diluted 1:2500 in assay buffer. After extensive washing, plates were developed by addition of 50µl/well NBT/B-CIP (Pierce Biotechnology) until development of spots was observed under the microscope. The reaction was stopped by washing plates thoroughly with distilled water. Plates were allowed to air-dry completely, and spots were counted using an automated ELISPOT reader.

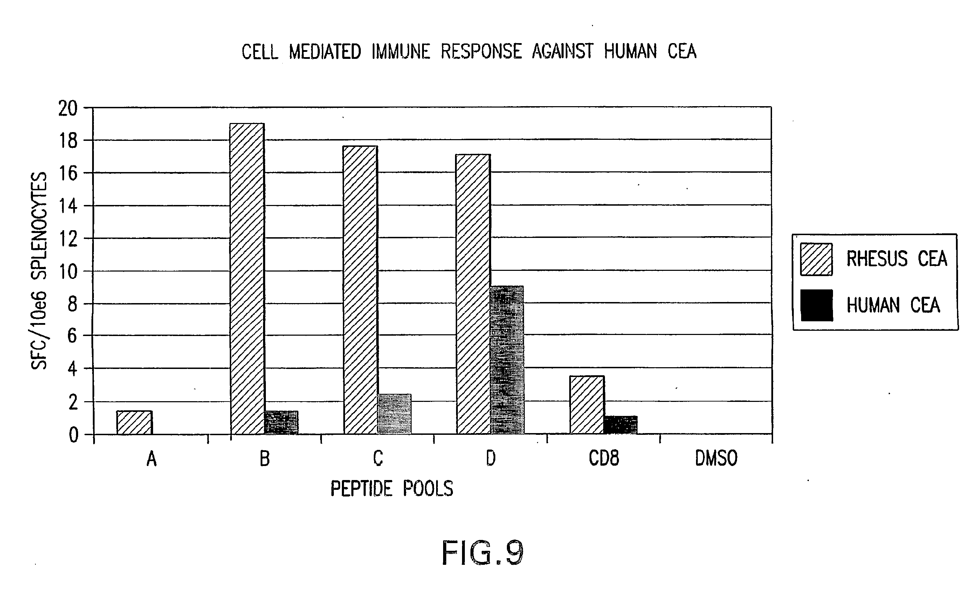

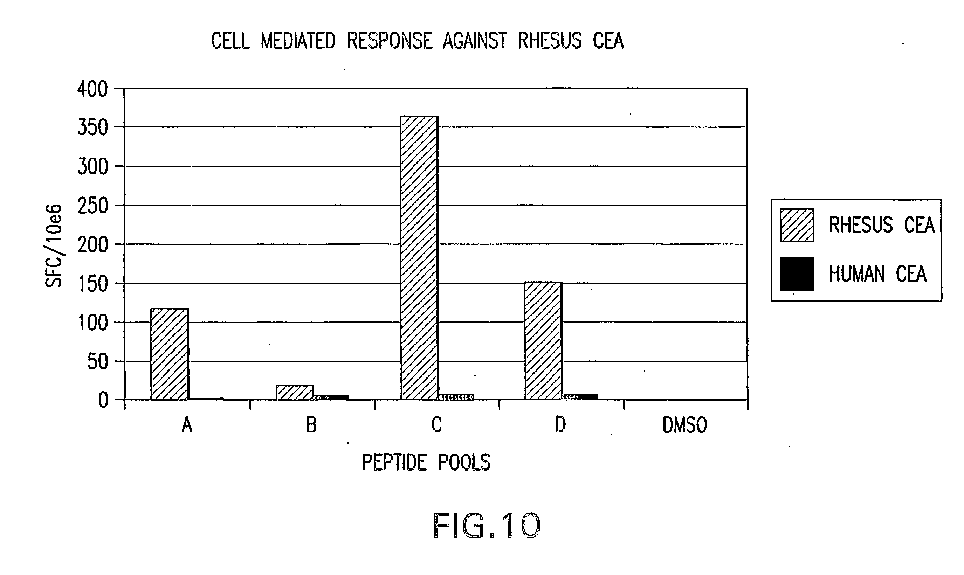

For cell mediated immune response, CEA.Tg mice were vaccinated either with hCEA expressing vectors or with rhCEA expressing vectors. Two groups were analyzed: the first group was analyzed by ELISPOT assay 21 days after last DNA injection, while the second group was boosted with 1x10¹⁰ vp of either Ad5-hCEA or Ad5-RhCEA and analyzed two weeks later. Results demonstrated that after four DNA injections, no significant cellular immune-response against hCEA was observed as measured by ELISPOT (not shown). On the other hand, mice that were boosted with Ad5 demonstrated a considerably increased response, consistent with breaking the immune-tolerance to CEA. This observation suggests that a useful vaccination protocol for the CEA self antigen would be the repeated administration of DNA by EGT, followed by an adenovirus boost (mixed modality). Importantly, immunization with rhesus CEA provided cross-reaction with human CEA peptides and vice-versa both in wild type and transgenic mice (data not shown). In particular, the immune response against human CEA was much better in transgenic mice using rhCEA as the immunogen (see FIGURE 9). These results show that a good response against CEA in transgenic mice could be obtained using the rhesus (xeno) gene. Response against rhesus CEA peptides is shown in FIGURE 10.

### EXAMPLE 10

### Immunization of Rhesus Macaques with rhCEA

In order to assess the efficiency of immunization of rhesus macaques *(macaca mulatta)* with the rhesus homologue of the human tumour antigen CEA, which is expressed in colorectal carcinomas, immunization studies were performed at the Biomedical Primate Research Centre (BPRC, Rijswijk, The Netherlands). Such immunization studies were designed to evaluate both B and T cell responses to immunization with the rhesus CEA antigen.

In this study (CV-1), 1 group of monkeys (consisting of 2 males and 2 females) was immunized with a plasmid DNA vector and adenovirus vector expressing rhesus CEACAM-5. For priming, animals were vaccinated intramuscularly with plasmid DNA expressing rhCEA at weeks 0, 4, 8, 12, and 16 by injection of DNA followed by electrical stimulation. The DNA injection consisted of a 1 ml solution (split over 2 sites with 0.5 ml/site) containing 5 mg plasmid DNA for animals weighing 2-5 kilos. Animals were injected under anesthesia (mixture of ketamine/xylazine).

For electrostimulation, 2 trains of 100 square bipolar pulses (1 sec each), were delivered every other second for a total treatment time of 3 sec. The pulse length was 2 msec/phase with a pulse frequency and amplitude of 100 Hz and 100 mA (constant current mode), respectively.

To measure the immune response to CEA using the above immunization protocol, blood samples were collected every four weeks. The cell mediated response was measured by IFNγ Elispot assay and the humoral response was measured by ELISA assay. Because no significant immune response was obtained at week 16, two further injections (week 24 and 28) were carried out using Ad5 expressing rhCEA. Upon Ad5 injection, a measurable immune response against rhCEA was detected for two monkeys (RI137 and CO12 covering peptide pool C and pool B + C, respectively. The cell mediated immune response began to decline in both monkeys at week 35.

The humoral immune response was followed over time upon DNA injection. Three monkeys (CO12, RI311 and RI002) showed a good anti-CEA antibody titer, ranging from 1:143 to 1:2099 and reaching a peak between weeks 12 and 16 after the first injection.

These data show that genetic vectors encoding rhCEA were able to break the immune tolerance to this tumor antigen in primates. Both cell mediated (50% of treated monkeys) and humoral (75% of treated monkeys) immunity were involved in the immune response.

### EXAMPLE 11

### Immunization of Rhesus Macaques with Rhesus Homologs of Human Tumor-Associated Antigens

A second series of immunization studies was performed in order to assess the efficiency of immunization of Rhesus macaques (*Macaca mulatta*) with rhesus homologues of the human tumor antigens HER2/neu, Ep-CAM and CEA, which are all expressed in colorectal carcinomas. Protocols were designed to evaluate both B and T cell responses to these tumor antigens in combination.

In this study, a second group of 4 rhesus monkeys (2 males and 2 females) were immunized with a mixture of three plasmid DNA vectors expressing the rhesus homologues of human tumor antigens Ep-CAM (pV1J-rhEpCAM), CEA pV1J-rhCEA), and HER2/neu (pV1J-rhHER2).

Animals were primed by intramuscular injection of plasmid DNA at weeks 0, 4, 8, 12, and 16, followed by electrostimulation. The DNA injection consisted of a 1 ml solution (split over 2 sites with 0.5 ml/site) containing 6 mg plasmid DNA for animals weighing 2-5 kilos. Animals were injected under anesthesia (mixture of ketamine/xylazine).

For electrostimulation, 2 trains of 100 square bipolar pulses (1 sec each), were delivered every other second for a total treatment time of 3 sec. The pulse length was 2 msec/phase with a pulse frequency and amplitude of 100 Hz and 100 mA (constant current mode), respectively.

The same group of animals was boosted by injection of a mixture of three Ad5-expressing rhesus CEA (Ad5-rhCEA), rhesus HER2/neu (Ad5-rhHER2), and rhesus EpCAM (Ad5-rhEpCAM). A total amount of 3x10exp11 viral particles (vp), were injected i.m. at weeks 23 and 27 (1x10exp11 vp for each of the three viruses).

To measure the immune response to the three tumor antigens using the above immunization protocol, blood samples were collected every four weeks. The cell mediated immune response was measured by IFN-γ+ ELISPOT assay, whereas the humoral response was measured by ELISA.

Monkeys RI449 and RI519 showed a detectable HER2-specific cell-mediated response, as measured by IFN-γ ELISPOT analysis. A similar analysis did not detect any significant response against rhCEA and rhEpCAM.

In a third study, 4 rhesus monkeys were immunized with a mixture of Ad5-rhHER2, Ad5-rhCEA and Ad5-rhEpCAM by i.m. injection of Ad5 derivatives at weeks 0, 2 and 4. A 1 ml solution (split over 2 sites with 0.5 ml/site) containing 3x10exp11vp (10exp11 for each of the three Ad5 virus) was administered to animals weighing 2-5 kilos, under anesthesia (mixture of ketamine/xylazine).

The cell mediated response was measured by IFNγ ELISPOT assay. For Her2/Neu, three out of four monkeys showed a detectable response. No significant cell mediated responses were measured for rhCEA and rhEpCAM.

In summary, the immunization protocol discussed above was effective in inducing a specific immune response against rhHER2/neu in rhesus monkeys. It is unclear why co-immunization with vectors carrying three different tumour antigens was not effective in inducing an innume response against rhCEA, as compared to study 1, which used only rhCEA as immunogen. Though not wishing to be bound by theory, it is possible that the expression of rhHER2/Neu and the presence of immunodominant epitopes limited the generation and the expansion of subdominant rhCEA specific T-cells.

## Claims

1. An isolated nucleic acid molecule, comprising a sequence of nucleotides that encodes a rhesus monkey carcinoembryonic antigen (rhCEA) protein as set forth in SEQ ID NO:2 or SEQ ID NO:8.

2. The isolated nucleic acid molecule of claim 1 wherein the nucleic acid is DNA.

3. The isolated nucleic acid molecule of claim 1 wherein the nucleic acid is mRNA.

4. The isolated nucleic acid molecule of claim 1 wherein the nucleic acid is cDNA.

5. The isolated nucleic acid molecule of claim 1 wherein the sequence of nucleotides comprises the sequence of nucleotides set forth in SEQ ID NO:1 or SEQ ID NO:5.

6. A vector comprising the nucleic acid molecule of claim 1.

7. A host cell comprising the vector of claim 6.

8. A process for expressing a rhesus carcinoembryonic antigen (rhCEA) protein in a recombinant host cell, comprising:
(a) introducing a vector comprising the nucleic acid of claim 1 into a suitable host cell; and,
(b) culturing the host cell under conditions which allow expression of said rhesus monkey CEA protein.

9. A process for expressing a rhesus monkey carcinoembryonic antigen (rhCEA) protein in a recombinant host cell, comprising:
(a) introducing a vector comprising the nucleic acid of claim 5 into a suitable host cell; and,
(b) culturing the host cell under conditions which allow expression of said rhesus monkey CEA protein.

10. An isolated and purified rhesus monkey carcinoembryonic antigen (rhCEA) protein comprising a sequence of amino acids as set forth in SEQ ID NO:2 or SEQ ID NO:8.

11. The use of a vaccine vector comprising an isolated nucleic acid molecule, the isolated nucleic acid molecule comprising a sequence of nucleotides that encodes a rhesus monkey carcinoembryonic antigen (rhCEA) protein as set forth in SEQ ID NO:2 or SEQ ID NO:8, for the manufacture of a medicament for preventing or treating cancer in a mammal.

12. Use according to claim 11 wherein the mammal is human.

13. Use according to claim 11 wherein the vector is an adenovirus vector or a plasmid vector.

14. Use according to claim 11 wherein the vector is an adenoviral vector comprising an adenoviral genome with a deletion in the adenovirus E1 region, and an insert in the adenovirus E1 region, wherein the insert comprises an expression cassette comprising:
(a) a polynucleotide encoding the rhesus monkey CEA protein; and
(b) a promoter operably linked to the polynucleotide.

15. Use according to claim 11 wherein the vector is a plasmid vaccine vector, which comprises a plasmid portion and an expressible cassette comprising
(a) a polynucleotide encoding the rhesus monkey CEA protein; and
(b) a promoter operably linked to the polynucleotide.

16. An adenovirus vaccine, vector comprising an adenoviral genome with a deletion in the E1 region, and an insert in the E1 region, wherein the insert comprises an expression cassette comprising:
(a) a polynucleotide encoding a rhesus monkey carcino embryonic antigen (rhCEA) protein as set forth in SEQ ID NO:2 or SEQ ID NO: 8 and
(b) a promoter operably linked to the polynucleotide.

17. An adenovirus vector according to claim 16 which is an Ad 5 or Ad 6 vector.

18. A vaccine plasmid comprising a plasmid portion and an expression cassette portion, the expression cassette portion comprising:
(a) a polynucleotide encoding a rhesus monkey carcino embryonic antigen (rhCEA) protein as set forth in SEQ ID NO:2 or SEQ ID NO: 8 and
(b) a promoter operably linked to the polynucleotide.

## Patentansprüche

1. Isoliertes Nukleinsäuremolekül, umfassend eine Nukleotidsequenz, welche für ein karzinoembryonales Rhesusaffen-Antigen (rhCEA)-Protein wie in SEQ-ID-Nr. 2 oder SEQ-ID-Nr. 8 angegeben codiert.

2. Isoliertes Nukleinsäuremolekül nach Anspruch 1, wobei die Nukleinsäure DNA ist.

3. Isoliertes Nukleinsäuremolekül nach Anspruch 1, wobei die Nukleinsäure mRNA ist.

4. Isoliertes Nukleinsäuremolekül nach Anspruch 1, wobei die Nukleinsäure cDNA ist.

5. Isoliertes Nukleinsäuremolekül nach Anspruch 1, wobei die Nukleotidsequenz die in SEQ-ID-Nr. 1 oder SEQ-ID-Nr. 5 angegebene Nukleotidsequenz umfasst.

6. Vektor, umfassend das Nukleinsäuremolekül nach Anspruch 1.

7. Wirtszelle, umfassend den Vektor nach Anspruch 6.

8. Verfahren zur Expression eines karzinoembryonalen Rhesusaffen-Antigen (rhCEA)-Proteins in einer rekombinanten Wirtszelle, umfassend:
a) Einführen eines Vektors, welcher die Nukleinsäure nach Anspruch 1 umfasst, in eine geeignete Wirtszelle; und
b) Kultivieren der Wirtszelle unter Bedingungen, welche die Expression des Rhesusaffen-CEA-Proteins erlauben.

9. Verfahren zur Expression eines karzinoembryonalen Rhesusaffen-Antigen (rhCEA)-Proteins in einer rekombinanten Wirtszelle, umfassend:
a) Einführen eines Vektors, welcher die Nukleinsäure nach Anspruch 5 umfasst, in eine geeignete Wirtszelle; und
b) Kultivieren der Wirtszelle unter Bedingungen, welche die Expression des Rhesusaffen-CEA-Proteins erlauben.

10. Isoliertes und gereinigtes karzinoembryonales Rhesusaffen-Antigen (rhCEA)-Protein, umfassend eine Aminosäuresequenz wie in SEQ-ID-Nr. 2 oder SEQ-ID-Nr. 8 angegeben.

11. Verwendung eines Vakzinvektors, umfassend ein isoliertes Nukleinsäuremolekül, wobei das isolierte Nukleinsäuremolekül eine Nukleotidsequenz umfasst, welche für ein karzinoembryonales Rhesusaffen-Antigen (rhCEA)-Protein wie in SEQ-ID-Nr. 2 oder SEQ-ID-Nr. 8 angegeben codiert, zur Herstellung eines Medikaments zur Verhütung oder Behandlung von Krebs bei einem Säuger.

12. Verwendung nach Anspruch 11, wobei der Säuger ein Mensch ist.

13. Verwendung nach Anspruch 11, wobei der Vektor ein Adenovirusvektor oder ein Plasmidvektor ist.

14. Verwendung nach Anspruch 11, wobei der Vektor ein Adenovirusvektor ist, welcher ein Adenovirusgenom mit einer Deletion in der Adenovirus-E1-Region und einem Insert in der Adenovirus-E1-Region umfasst, wobei das Insert umfasst eine Expressionskassette, umfassend:
a) ein Polynukleotid, codierend für das Rhesusaffen-CEA-Protein; und
b) einen Promotor in funktionsfähiger Verknüpfung mit dem Polynukleotid.

15. Verwendung nach Anspruch 11, wobei der Vektor ein Plasmid-Vakzinvektor ist, welcher umfasst einen Plasmidanteil und eine exprimierbare Kassette, umfassend:
a) ein Polynukleotid, codierend für das Rhesusaffen-CEA-Protein; und
b) einen Promotor in funktionsfähiger Verknüpfung mit dem Polynukleotid.

16. Adenovirus-Vakzinvektor, umfassend ein Adenovirusgenom mit einer Deletion in der E1-Region und einem Insert in der E1-Region, wobei das Insert umfasst eine Expressionskassette, umfassend:
a) ein Polynukleotid, codierend für ein karzinoembryonales Rhesusaffen-Antigen (rhCEA)-Protein wie in SEQ-ID-Nr. 2 oder SEQ-ID-Nr. 8 angegeben; und
b) einen Promotor in funktionsfähiger Verknüpfung mit dem Polynukleotid.

17. Adenovirusvektor nach Anspruch 16, welcher ein Ad5- oder Ad6-Vektor ist.

18. Vakzinplasmid, umfassend einen Plasmidanteil und einen Expressionskassettenanteil, wobei der Expressionskassettenanteil umfasst:
a) ein Polynukleotid, codierend für ein karzinoembryonales Rhesusaffen-Antigen (rhCEA)-Protein wie in SEQ-ID-Nr. 2 oder SEQ-ID-Nr. 8 angegeben; und
b) einen Promotor in funktionsfähiger Verknüpfung mit dem Polynukleotid.

## Revendications

1. Molécule d'acide nucléique isolée, comprenant une séquence de nucléotides qui code pour une protéine d'antigène carcino-embryonnaire de singe rhésus (rhCEA) telle que représentée dans la SEQ ID n° : 2 ou la SEQ ID n° : 8.

2. Molécule d'acide nucléique isolée selon la revendication 1, dans laquelle l'acide nucléique est un ADN.

3. Molécule d'acide nucléique isolée selon la revendication 1, dans laquelle l'acide nucléique est un ARNm.

4. Molécule d'acide nucléique isolée selon la revendication 1, dans laquelle l'acide nucléique est un ADNc.

5. Molécule d'acide nucléique isolée selon la revendication 1, dans laquelle la séquence de nucléotides comprend la séquence de nucléotides représentée dans la SEQ ID n° : 1 ou la SEQ ID n° : 5.

6. Vecteur comprenant la molécule d'acide nucléique de la revendication 1.

7. Cellule hôte comprenant le vecteur de la revendication 6.

8. Procédé pour l'expression d'une protéine d'antigène carcino-embryonnaire de singe rhésus (rhCEA) dans une cellule hôte recombinante, comprenant :
(a) l'introduction d'un vecteur comprenant l'acide nucléique de la revendication 1 dans une cellule hôte convenable ; et
(b) la culture de la cellule hôte dans des conditions qui permettent l'expression de ladite protéine CEA du singe rhésus.

9. Procédé pour l'expression d'une protéine d'antigène carcino-embryonnaire de singe rhésus (rhCEA) dans une cellule hôte recombinante, comprenant :
(a) l'introduction d'un vecteur comprenant l'acide nucléique de la revendication 5 dans une cellule hôte convenable ; et
(b) la culture de la cellule hôte dans des conditions qui permettent l'expression de ladite protéine CEA du singe rhésus.

10. Protéine d'antigène carcino-embryonnaire de singe rhésus (rhCEA) isolée et purifiée comprenant une séquence d'acides aminés représentée dans la SEQ ID n° : 2 ou la SEQ ID n° : 8.

11. Utilisation d'un vecteur de vaccin comprenant un acide nucléique isolé, la molécule d'acide nucléique isolée comprenant une séquence de nucléotides qui code pour une protéine d'antigène carcino-embryonnaire de singe rhésus (rhCEA) telle que représentée dans la SEQ ID n° : 2 ou la SEQ ID n° : 8, pour la fabrication d'un médicament pour la prévention ou le traitement du cancer chez un mammifère.

12. Utilisation selon la revendication 11, dans laquelle le mammifère est un être humain.

13. Utilisation selon la revendication 11, dans laquelle le vecteur est un vecteur à adénovirus ou un vecteur plasmidique.

14. Utilisation selon la revendication 11, dans laquelle le vecteur est un vecteur à adénovirus comprenant un génome d'adénovirus avec une délétion dans la région E1 de l'adénovirus, et un insert dans la région E1 de l'adénovirus, dans lequel l'insert comprend une cassette d'expression comprenant :
(a) un polynucléotide codant pour la protéine CEA du singe rhésus ; et
(b) un promoteur lié de façon opérationnelle au polynucléotide.

15. Utilisation selon la revendication 11, dans laquelle le vecteur est un vecteur de vaccin plasmidique qui comprend une partie plasmidique et une cassette d'expression comprenant :
(a) un polynucléotide codant pour la protéine CEA du singe rhésus ; et
(b) un promoteur lié de façon opérationnelle au polynucléotide.

16. Vecteur de vaccin à adénovirus comprenant un génome d'adénovirus avec une délétion dans la région E1, et un insert dans la région E1, dans lequel l'insert comprend une cassette d'expression comprenant :
(a) un polynucléotide codant pour la protéine d'antigène carcino-embryonnaire de singe rhésus (rhCEA) telle que représentée dans la SEQ ID n° : 2 ou la SEQ ID n° : 8 ; et
(b) un promoteur lié de façon opérationnelle au polynucléotide.

17. Vecteur à adénovirus selon la revendication 16, qui est un vecteur Ad 5 ou Ad 6.

18. Plasmide de vaccin comprenant une partie plasmidique et une partie de cassette d'expression la partie de cassette d'expression comprenant :
(a) un polynucléotide codant pour la protéine d'antigène carcino-embryonnaire de singe rhésus (rhCEA) telle que représentée dans la SEQ ID n° : 2 ou la SEQ ID n° : 8 ; et
(b) un promoteur lié de façon opérationnelle au polynucléotide.


### SEQUENCE LISTING

<110> Luigi Aurisicchio
   Fabio Palombo
   Paolo Monaci
   Nicola La Monica
   Gennaro Ciliberto
   Armin Lahm
<120> RHESUS CARCINO EMBRYONIC ANTIGEN,
   NUCLEOTIDES ENCODING SAME, AND USES THEREOF
<130> ITR0045 PCT
<150> 60/447,203
   <151> 2003-02-13
<160> 16
<170> FastSEQ for Windows Version 4.0
<210> 1
   <211> 2118
   <212> DNA
   <213> Macaca mulatta
<400> 1
   atggggtctc cctcagcccc tcttcacaga tggtgcatcc cctggcagac gctcctgctc 60
   acagcctcac ttctaacctt ctggaacccg cccaccactg cccagctcac tattgaatcc 120
   aggccgttca atgttgcaga ggggaaggag gttcttctac ttgcccacaa tgtgtcccag 180
   aatctttttg gctacatttg gtacaaggga gaaagagtgg atgccagccg tcgaattgga 240
   tcatgtgtaa taagaactca acaaattacc ccagggcccg cacacagcgg tcgagagaca 300
   atagacttca atgcatccct gctgatccac aatgtcaccc agagtgacac aggatcctac 360
   accatacaag tcataaagga agatcttgtg aatgaagaag caactggcca gttccgggta 420
   tacccggagc tgcccaagcc ctacatctcc agcaacaact ccaaccccgt ggaggacaag 480
   gatgctgtgg ccttaacctg tgaacctgag actcaggaca caacctacct gtggtgggta 540
   aacaatcaga gcctcccggt cagtcccagg ctggagctgt ccagtgacaa caggaccctc 600
   actgtattca atattccaag aaatgacaca acatcctaca aatgtgaaac ccagaaccca 660
   gtgagtgtca gacgcagcga cccagtcacc ctgaacgtcc tctatggccc ggatgcgccc 720
   accatttccc ctctaaacac accttacaga gcaggggaaa atctgaacct cacctgccac 780
   gcagcctcta acccaactgc acagtacttt tggtttgtca atgggacgtt ccagcaatcc 840
   acacaagagc tctttatacc caacatcacc gtgaataata gcggatccta tatgtgccaa 900
   gcccataact cagccactgg cctcaatagg accacagtca cggcgatcac agtctacgcg 960
   gagctgccca agccctacat caccagcaac aactccaacc ccatagagga caaggatgct 1020
   gtgaccttaa cctgtgaacc tgagactcag gacacaacct acctgtggtg ggtaaacaat 1080
   cagagcctct cggtcagttc caggctggag ctgtccaatg acaacaggac cctcactgta 1140
   ttcaatattc caagaaacga cacaacgttc tacgaatgtg agacccagaa cccagtgagt 1200
   gtcagacgca gcgacccagt caccctgaat gtcctctatg gcccggatgc gcccaccatt 1260
   tcccctctaa acacacctta cagagcaggg gaaaatctga acctctcctg ccacgcagcc 1320
   tctaacccag ctgcacagta ctcttggttt gtcaatggga cgttccagca atccacacaa 1380
   gagctcttta tacccaacat caccgtgaat aatagcggat cctatatgtg ccaagcccat 1440
   aactcagcca ctggcctcaa taggaccaca gtcacggcga tcacagtcta tgtggagctg 1500
   cccaagccct acatctccag caacaactcc aaccccatag aggacaagga tgctgtgacc 1560
   ttaacctgtg aacctgtggc tgagaacaca acctacctgt ggtgggtaaa caatcagagc 1620
   ctctcggtca gtcccaggct gcagctctcc aatggcaaca ggatcctcac tctactcagt 1680
   gtcacacgga atgacacagg accctatgaa tgtggaatcc agaactcaga gagtgcaaaa 1740
   cgcagtgacc cagtcaccct gaatgtcacc tatggcccag acacccccat catatccccc 1800
   ccagacttgt cttaccgttc gggagcaaac ctcaacctct cctgccactc ggactctaac 1860
   ccatccccgc agtattcttg gcttatcaat gggacactgc ggcaacacac acaagttctc 1920
   tttatctcca aaatcacatc aaacaatagc ggggcctatg cctgttttgt ctctaacttg 1980
   gctaccggtc gcaataactc catagtcaag aacatctcag tctcctctgg cgattcagca 2040
   cctggaagtt ctggtctctc agctagggct actgtcggca tcataattgg aatgctggtt 2100
   ggggttgctc tgatgtag 2118
<210> 2
   <211> 705
   <212> PRT
   <213> Macaca mulatta
<400> 2
   Met Gly Ser Pro Ser Ala Pro Leu His Arg Trp Cys Ile Pro Trp Gln
   1 5 10 15
   Thr Leu Leu Leu Thr Ala Ser Leu Leu Thr Phe Trp Asn Pro Pro Thr
   20 25 30
   Thr Ala Gln Leu Thr Ile Glu Ser Arg Pro Phe Asn Val Ala Glu Gly
   35 40 45
   Lys Glu Val Leu Leu Leu Ala His Asn Val Ser Gln Asn Leu Phe Gly
   50 55 60
   Tyr Ile Trp Tyr Lys Gly Glu Arg Val Asp Ala Ser Arg Arg Ile Gly
   65 70 75 80
   Ser Cys Val Ile Arg Thr Gln Gln Ile Thr Pro Gly Pro Ala His Ser
   85 90 95
   Gly Arg Glu Thr Ile Asp Phe Asn Ala Ser Leu Leu Ile His Asn Val
   100 105 110
   Thr Gln Ser Asp Thr Gly Ser Tyr Thr Ile Gln Val Ile Lys Glu Asp
   115 120 125
   Leu Val Asn Glu Glu Ala Thr Gly Gln Phe Arg Val Tyr Pro Glu Leu
   130 135 140
   Pro Lys Pro Tyr Ile Ser Ser Asn Asn Ser Asn Pro Val Glu Asp Lys
   145 150 155 160
   Asp Ala Val Ala Leu Thr Cys Glu Pro Glu Thr Gln Asp Thr Thr Tyr
   165 170 175
   Leu Trp Trp Val Asn Asn Gln Ser Leu Pro Val Ser Pro Arg Leu Glu
   180 185 190
   Leu Ser Ser Asp Asn Arg Thr Leu Thr Val Phe Asn Ile Pro Arg Asn
   195 200 205
   Asp Thr Thr Ser Tyr Lys Cys Glu Thr Gln Asn Pro Val Ser Val Arg
   210 215 220
   Arg Ser Asp Pro Val Thr Leu Asn Val Leu Tyr Gly Pro Asp Ala Pro
   225 230 235 240
   Thr Ile Ser Pro Leu Asn Thr Pro Tyr Arg Ala Gly Glu Asn Leu Asn
   245 250 255
   Leu Thr Cys His Ala Ala Ser Asn Pro Thr Ala Gln Tyr Phe Trp Phe
   260 265 270
   Val Asn Gly Thr Phe Gln Gln Ser Thr Gln Glu Leu Phe Ile Pro Asn
   275 280 285
   Ile Thr Val Asn Asn Ser Gly Ser Tyr Met Cys Gln Ala His Asn Ser
   290 295 300
   Ala Thr Gly Leu Asn Arg Thr Thr Val Thr Ala Ile Thr Val Tyr Ala
   305 310 315 320
   Glu Leu Pro Lys Pro Tyr Ile Thr Ser Asn Asn Ser Asn Pro Ile Glu
   325 330 335
   Asp Lys Asp Ala Val Thr Leu Thr Cys Glu Pro Glu Thr Gln Asp Thr
   340 345 350
   Thr Tyr Leu Trp Trp Val Asn Asn Gln Ser Leu Ser Val Ser Ser Arg
   355 360 365
   Leu Glu Leu Ser Asn Asp Asn Arg Thr Leu Thr Val Phe Asn Ile Pro
   370 375 380
   Arg Asn Asp Thr Thr Phe Tyr Glu Cys Glu Thr Gln Asn Pro Val Ser
   385 390 395 400
   Val Arg Arg Ser Asp Pro Val Thr Leu Asn Val Leu Tyr Gly Pro Asp
   405 410 415
   Ala Pro Thr Ile Ser Pro Leu Asn Thr Pro Tyr Arg Ala Gly Glu Asn
   420 425 430
   Leu Asn Leu Ser Cys His Ala Ala Ser Asn Pro Ala Ala Gln Tyr Ser
   435 440 445
   Trp Phe Val Asn Gly Thr Phe Gln Gln Ser Thr Gln Glu Leu Phe Ile
   450 455 460
   Pro Asn Ile Thr Val Asn Asn Ser Gly Ser Tyr Met Cys Gln Ala His
   465 470 475 480
   Asn Ser Ala Thr Gly Leu Asn Arg Thr Thr Val Thr Ala Ile Thr Val
   485 490 495
   Tyr Val Glu Leu Pro Lys Pro Tyr Ile Ser Ser Asn Asn Ser Asn Pro
   500 505 510
   Ile Glu Asp Lys Asp Ala Val Thr Leu Thr Cys Glu Pro Val Ala Glu
   515 520 525
   Asn Thr Thr Tyr Leu Trp Trp Val Asn Asn Gln Ser Leu Ser Val Ser
   530 535 540
   Pro Arg Leu Gln Leu Ser Asn Gly Asn Arg Ile Leu Thr Leu Leu Ser
   545 550 555 560
   Val Thr Arg Asn Asp Thr Gly Pro Tyr Glu Cys Gly Ile Gln Asn Ser
   565 570 575
   Glu Ser Ala Lys Arg Ser Asp Pro Val Thr Leu Asn Val Thr Tyr Gly
   580 585 590
   Pro Asp Thr Pro Ile Ile Ser Pro Pro Asp Leu Ser Tyr Arg Ser Gly
   595 600 605
   Ala Asn Leu Asn Leu Ser Cys His Ser Asp Ser Asn Pro Ser Pro Gln
   610 615 620
   Tyr Ser Trp Leu Ile Asn Gly Thr Leu Arg Gln His Thr Gln Val Leu
   625 630 635 640
   Phe Ile Ser Lys Ile Thr Ser Asn Asn Ser Gly Ala Tyr Ala Cys Phe
   645 650 655
   Val Ser Asn Leu Ala Thr Gly Arg Asn Asn Ser Ile Val Lys Asn Ile
   660 665 670
   Ser Val Ser Ser Gly Asp Ser Ala Pro Gly Ser Ser Gly Leu Ser Ala
   675 680 685
   Arg Ala Thr Val Gly Ile Ile Ile Gly Met Leu Val Gly Val Ala Leu
   690 695 700
   Met
   705
<210> 3
   <211> 32
   <212> DNA
   <213> Artificial Sequence
<220>
   <223> PCR Primer
<221> misc_feature
   <222> (0)...(0)
   <223> S = C or G
<221> misc_feature
   <222> (0)...(0)
   <223> R = A or G
<400> 3
   ccgaattccg gacasagcag rcagcagrsa cc 32
<210> 4
   <211> 37
   <212> DNA
   <213> Artificial Sequence
<220>
   <223> PCR Primer
<400> 4
   ccgctcgagc ggctgctaca tcagagcaac cccaacc 37
<210> 5
   <211> 2118
   <212> DNA
   <213> Macaca mulatta
<400> 5
   atggggtctc cctcagcccc tcttcacaga tggtgcatcc cctggcagac gctcctgctc 60
   acagcctcac ttctaacctt ctggaacccg cccaccactg cccagctcac tattgaatcc 120
   aggccgttca atgttgcaga ggggaaggag gttcttctac ttgcccacaa tgtgtcccag 180
   aatctttttg gctacatttg gtacaaggga gaaagagtgg atgccagccg tcgaattgga 240
   tcatgtgtaa taagaactca acaaattacc ccagggcccg cacacagcgg tcgagagaca 300
   atagacttca atgcatccct gctgatccac aatgtcaccc agagtgacac aggatcctac 360
   accatacaag tcataaagga agatcttgtg aatgaagaag caactggcca gttccgggta 420
   tacccggagc tgcccaagcc ctacatctcc agcaacaact ccaaccccgt ggaggacaag 480
   gatgctgtgg ccttaacctg tgaacctgag actcaggaca caacctacct gtggtgggta 540
   aacaatcaga gcctcccggt cagtcccagg ctggagctgt ccagtgacaa caggaccctc 600
   actgtattca atattccaag aaatgacaca acatcctaca aatgtgaaac ccagaaccca 660
   gtgagtgtca gacgcagcga cccagtcacc ctgaacgtcc tctatggccc ggatgcgccc 720
   accatttccc ctctaaacac accttacaga gcaggggaaa atctgaacct cacctgccac 780
   gcagcctcta acccaactgc acagtacttt tggtttgtca atgggacgtt ccagcaatcc 840
   acacaagagc tctttatacc caacatcacc gtgaataata gcggatccta tatgtgccaa 900
   gcccataact cagccactgg cctcaatagg accacagtca cggcgatcac agtctacgcg 960
   gagctgccca agccctacat caccagcaac aactccaacc ccatagagga caaggatgct 1020
   gtgaccttaa cctgtgaacc tgagactcag gacacaacct acctgtggtg ggtaaacaat 1080
   cagagcctct cggtcagttc caggctggag ctgtccaatg acaacaggac cctcactgta 1140
   ttcaatattc caagaaacga cacaacgttc tacgaatgtg agacccagaa cccagtgagt 1200
   gtcagacgca gcgacccagt caccctgaat gtcctctatg gcccggatgc gcccaccatt 1260
   tcccctctaa acacacctta cagagcaggg gaaaatctga acctctcctg ccacgcagcc 1320
   tctaacccag ctgcacagta cttttggttt gtcaatggga cgttccagca atccacacaa 1380
   gagctcttta tacccaacat caccgtgaat aatagcggat cctatatgtg ccaagcccat 1440
   aactcagcca ctggcctcaa taggaccaca gtcacggcga tcacagtcta tgtggagctg 1500
   cccaagccct acatctccag caacaactcc aaccccatag aggacaagga tgctgtgacc 1560
   ttaacctgtg aacctgtggc tgagaacaca acctacctgt ggtgggtaaa caatcagagc 1620
   ctctcggtca gtcccaggct gcagctctcc aatggcaaca ggatcctcac tctactcagt 1680
   gtcacacgga atgacacagg accctatgaa tgtggaatcc agaactcaga gagtgcaaaa 1740
   cgcagtgacc cagtcaccct gaatgtcacc tatggcccag acacccccat catatccccc 1800
   ccagacttgt cttaccgttc gggagcaaac ctcaacctct cctgccactc ggactctaac 1860
   ccatccccgc agtattcttg gcttatcaat gggacactgc ggcaacacac acaagttctc 1920
   tttatctcca aaatcacatc aaacaataac ggggcctatg cctgttttgt ctctaacttg 1980
   gctaccggtc gcaataactc catagtcaag aacatctcag tctcctctgg cgattcagca 2040
   cctggaagtt ctggtctctc agctagggct actgtcggca tcataattgg aatgctggtt 2100
   ggggttgctc tgatgtag 2118
<210> 6
   <211> 2109
   <212> DNA
   <213> Homo sapiens
<400> 6
   atggagtctc cctcggcccc tccccacaga tggtgcatcc cctggcagag gctcctgctc 60
   acagcctcac ttctaacctt ctggaacccg cccaccactg ccaagctcac tattgaatcc 120
   acgccgttca atgtcgcaga ggggaaggag gtgcttctac ttgtccacaa tctgccccag 180
   catctttttg gctacagctg gtacaaaggt gaaagagtgg atggcaaccg tcaaattata 240
   ggatatgtaa taggaactca acaagctacc ccagggcccg catacagtgg tcgagagata 300
   atatacccca atgcatccct gctgatccag aacatcatcc agaatgacac aggattctac 360
   accctacacg tcataaagtc agatcttgtg aatgaagaag caactggcca gttccgggta 420
   tacccggagc tgcccaagcc ctccatctcc agcaacaact ccaaacccgt ggaggacaag 480
   gatgctgtgg ccttcacctg tgaacctgag actcaggacg caacctacct gtggtgggta 540
   aacaatcaga gcctcccggt cagtcccagg ctgcagctgt ccaatggcaa caggaccctc 600
   actctattca atgtcacaag aaatgacaca gcaagctaca aatgtgaaac ccagaaccca 660
   gtgagtgcca ggcgcagtga ttcagtcatc ctgaatgtcc tctatggccc ggatgccccc 720
   accatttccc ctctaaacac atcttacaga tcaggggaaa atctgaacct ctcctgccat 780
   gcagcctcta acccacctgc acagtactct tggtttgtca atgggacttt ccagcaatcc 840
   acccaagagc tctttatccc caacatcact gtgaataata gtggatccta tacgtgccaa 900
   gcccataact cagacactgg cctcaatagg accacagtca cgacgatcac agtctatgca 960
   gagccaccca aacccttcat caccagcaac aactccaacc ccgtggagga tgaggatgct 1020
   gtagccttaa cctgtgaacc tgagattcag aacacaacct acctgtggtg ggtaaataat 1080
   cagagcctcc cggtcagtcc caggctgcag ctgtccaatg acaacaggac cctcactcta 1140
   ctcagtgtca caaggaatga tgtaggaccc tatgagtgtg gaatccagaa cgaattaagt 1200
   gttgaccaca gcgacccagt catcctgaat gtcctctatg gcccagacga ccccaccatt 1260
   tccccctcat acacctatta ccgtccaggg gtgaacctca gcctctcctg ccatgcagcc 1320
   tctaacccac ctgcacagta ttcttggctg attgatggga acatccagca acacacacaa 1380
   gagctcttta tctccaacat cactgagaag aacagcggac tctatacctg ccaggccaat 1440
   aactcagcca gtggccacag caggactaca gtcaagacaa tcacagtctc tgcggagctg 1500
   cccaagccct ccatctccag caacaactcc aaacccgtgg aggacaagga tgctgtggcc 1560
   ttcacctgtg aacctgaggc tcagaacaca acctacctgt ggtgggtaaa tggtcagagc 1620
   ctcccagtca gtcccaggct gcagctgtcc aatggcaaca ggaccctcac tctattcaat 1680
   gtcacaagaa atgacgcaag agcctatgta tgtggaatcc agaactcagt gagtgcaaac 1740
   cgcagtgacc cagtcaccct ggatgtcctc tatgggccgg acacccccat catttccccc 1800
   ccagactcgt cttacctttc gggagcgaac ctcaacctct cctgccactc ggcctctaac 1860
   ccatccccgc agtattcttg gcgtatcaat gggataccgc agcaacacac acaagttctc 1920
   tttatcgcca aaatcacgcc aaataataac gggacctatg cctgttttgt ctctaacttg 1980
   gctactggcc gcaataattc catagtcaag agcatcacag tctctgcatc tggaacttct 2040
   cctggtctct cagctggggc cactgtcggc atcatgattg gagtgctggt tggggttgct 2100
   ctgatatag 2109
<210> 7
   <211> 708
   <212> PRT
   <213> Homo sapiens
<400> 7
   Met Glu Ser Pro Ser Ala Pro Pro His Arg Trp Cys Ile Pro Trp Gln
   1 5 10 15
   Arg Leu Leu Leu Thr Ala Ser Leu Leu Thr Phe Trp Asn Pro Pro Thr
   20 25 30
   Thr Ala Lys Leu Thr Ile Glu Ser Thr Pro Phe Asn Val Ala Glu Gly
   35 40 45
   Lys Glu Val Leu Leu Leu Val His Asn Leu Pro Gln His Leu Phe Gly
   50 55 60
   Tyr Ser Trp Tyr Lys Gly Glu Arg Val Asp Gly Asn Arg Gln Ile Ile
   65 70 75 80
   Gly Tyr Val Ile Gly Thr Gln Gln Ala Thr Pro Gly Pro Ala Tyr Ser
   85 90 95
   Gly Arg Glu Ile Ile Tyr Pro Asn Ala Ser Leu Leu Ile Gln Asn Ile
   100 105 110
   Ile Gln Asn Asp Thr Gly Phe Tyr Thr Leu His Val Ile Lys Ser Asp
   115 120 125
   Leu Val Asn Glu Glu Ala Thr Gly Gln Phe Arg Val Tyr Pro Glu Leu
   130 135 140
   Pro Lys Pro Ser Ile Ser Ser Asn Asn Ser Lys Pro Val Glu Asp Lys
   145 150 155 160
   Asp Ala Val Ala Phe Thr Cys Glu Pro Glu Thr Gln Asp Ala Thr Tyr
   165 170 175
   Leu Trp Trp Val Asn Asn Gln Ser Leu Pro Val Ser Pro Arg Leu Gln
   180 185 190
   Leu Ser Asn Gly Asn Arg Thr Leu Thr Leu Phe Asn Val Thr Arg Asn
   195 200 205
   Asp Thr Ala Ser Tyr Lys Cys Glu Thr Gln Asn Pro Val Ser Ala Arg
   210 215 220
   Arg Ser Asp Ser Val Ile Leu Asn Val Leu Tyr Gly Pro Asp Ala Pro
   225 230 235 240
   Thr Ile Ser Pro Leu Asn Thr Ser Tyr Arg Ser Gly Glu Asn Leu Asn
   245 250 255
   Leu Ser Cys His Ala Ala Ser Asn Pro Pro Ala Gln Tyr Ser Trp Phe
   260 265 270
   Val Asn Gly Thr Phe Gln Gln Ser Thr Gln Glu Leu Phe Ile Pro Asn
   275 280 285
   Ile Thr Val Asn Asn Ser Gly Ser Tyr Thr Cys Gln Ala His Asn Ser
   290 295 300
   Asp Thr Gly Leu Asn Arg Thr Thr Val Thr Thr Ile Thr Val Tyr Ala
   305 310 315 320
   Glu Pro Pro Lys Pro Phe Ile Thr Ser Asn Asn Ser Asn Pro Val Glu
   325 330 335
   Asp Glu Asp Ala Val Ala Leu Thr Cys Glu Pro Glu Ile Gln Asn Thr
   340 345 350
   Thr Tyr Leu Trp Trp Val Asn Asn Gln Ser Leu Pro Val Ser Pro Arg
   355 360 365
   Leu Gln Leu Ser Asn Asp Asn Arg Thr Leu Thr Leu Leu Ser Val Thr
   370 375 380
   Arg Asn Asp Val Gly Pro Tyr Glu Cys Gly Ile Gln Asn Glu Leu Ser
   385 390 395 400
   Val Asp His Ser Asp Pro Val Ile Leu Asn Val Leu Tyr Gly Pro Asp
   405 410 415
   Asp Pro Thr Ile Ser Pro Ser Tyr Thr Tyr Tyr Arg Pro Gly Val Asn
   420 425 430
   Leu Ser Leu Ser Cys His Ala Ala Ser Asn Pro Pro Ala Gln Tyr Ser
   435 440 445
   Trp Leu Ile Asp Gly Asn Ile Gln Gln His Thr Gln Glu Leu Phe Ile
   450 455 460
   Ser Asn Ile Thr Glu Lys Asn Ser Gly Leu Tyr Thr Cys Gln Ala Asn
   465 470 475 480
   Asn Ser Ala Ser Gly His Ser Arg Thr Thr Val Lys Thr Ile Thr Val
   485 490 495
   Ser Ala Glu Leu Pro Lys Pro Ser Ile Ser Ser Asn Asn Ser Lys Pro
   500 505 510
   Val Glu Asp Lys Asp Ala Val Ala Phe Thr Cys Glu Pro Glu Ala Gln
   515 520 525
   Asn Thr Thr Tyr Leu Trp Trp Val Asn Gly Gln Ser Leu Pro Val Ser
   530 535 540
   Pro Arg Leu Gln Leu Ser Asn Gly Asn Arg Thr Leu Thr Leu Phe Asn
   545 550 555 560
   Val Thr Arg Asn Asp Ala Arg Ala Tyr Val Cys Gly Ile Gln Asn Ser
   565 570 575
   Val Ser Ala Asn Arg Ser Asp Pro Val Thr Leu Asp Val Leu Tyr Gly
   580 585 590
   Pro Asp Thr Pro Ile Ile Ser Pro Pro Asp Ser Ser Tyr Leu Ser Gly
   595 600 605
   Ala Asn Leu Asn Leu Ser Cys His Ser Ala Ser Asn Pro Ser Pro Gln
   610 615 620
   Tyr Ser Trp Arg Ile Asn Gly Ile Pro Gln Gln His Thr Gln Val Leu
   625 630 635 640
   Phe Ile Ala Lys Ile Thr Pro Asn Asn Asn Gly Thr Tyr Ala Cys Phe
   645 650 655
   Val Ser Asn Leu Ala Thr Gly Arg Asn Asn Ser Ile Val Lys Ser Ile
   660 665 670
   Thr Val Ser Ala Ser Gly Thr Ser Pro Gly Leu Ser Ala Gly Ala Thr
   675 680 685
   Val Gly Ile Met Ile Gly Val Leu Val Gly Val Ala Leu Ile Ser Glu
   690 695 700
   Gln Ile Asp Asn
   705
<210> 8
   <211>705
   <212> PRT
   <213> Macaca mulatta
<400> 8
   Met Gly Ser Pro Ser Ala Pro Leu His Arg Trp Cys Ile Pro Trp Gln
   1 5 10 15
   Thr Leu Leu Leu Thr Ala Ser Leu Leu Thr Phe Trp Asn Pro Pro Thr
   20 25 30
   Thr Ala Gln Leu Thr Ile Glu Ser Arg Pro Phe Asn Val Ala Glu Gly
   35 40 45
   Lys Glu Val Leu Leu Leu Ala His Asn Val Ser Gln Asn Leu Phe Gly
   50 55 60
   Tyr Ile Trp Tyr Lys Gly Glu Arg Val Asp Ala Ser Arg Arg Ile Gly
   65 70 75 80
   Ser Cys Val Ile Arg Thr Gln Gln Ile Thr Pro Gly Pro Ala His Ser
   85 90 95
   Gly Arg Glu Thr Ile Asp Phe Asn Ala Ser Leu Leu Ile His Asn Val
   100 105 110
   Thr Gln Ser Asp Thr Gly Ser Tyr Thr Ile Gln Val Ile Lys Glu Asp
   115 120 125
   Leu Val Asn Glu Glu Ala Thr Gly Gln Phe Arg Val Tyr Pro Glu Leu
   130 135 140
   Pro Lys Pro Tyr Ile Ser Ser Asn Asn Ser Asn Pro Val Glu Asp Lys
   145 150 155 160
   Asp Ala Val Ala Leu Thr Cys Glu Pro Glu Thr Gln Asp Thr Thr Tyr
   165 170 175
   Leu Trp Trp Val Asn Asn Gln Ser Leu Pro Val Ser Pro Arg Leu Glu
   180 185 190
   Leu Ser Ser Asp Asn Arg Thr Leu Thr Val Phe Asn Ile Pro Arg Asn
   195 200 205
   Asp Thr Thr Ser Tyr Lys Cys Glu Thr Gln Asn Pro Val Ser Val Arg
   210 215 220
   Arg Ser Asp Pro Val Thr Leu Asn Val Leu Tyr Gly Pro Asp Ala Pro
   225 230 235 240
   Thr Ile Ser Pro Leu Asn Thr Pro Tyr Arg Ala Gly Glu Asn Leu Asn
   245 250 255
   Leu Thr Cys His Ala Ala Ser Asn Pro Thr Ala Gln Tyr Phe Trp Phe
   260 265 270
   Val Asn Gly Thr Phe Gln Gln Ser Thr Gln Glu Leu Phe Ile Pro Asn
   275 280 285
   Ile Thr Val Asn Asn Ser Gly Ser Tyr Met Cys Gln Ala His Asn Ser
   290 295 300
   Ala Thr Gly Leu Asn Arg Thr Thr Val Thr Ala Ile Thr Val Tyr Ala
   305 310 315 320
   Glu Leu Pro Lys Pro Tyr Ile Thr Ser Asn Asn Ser Asn Pro Ile Glu
   325 330 335
   Asp Lys Asp Ala Val Thr Leu Thr Cys Glu Pro Glu Thr Gln Asp Thr
   340 345 350
   Thr Tyr Leu Trp Trp Val Asn Asn Gln Ser Leu Ser Val Ser Ser Arg
   355 360 365
   Leu Glu Leu Ser Asn Asp Asn Arg Thr Leu Thr Val Phe Asn Ile Pro
   370 375 380
   Arg Asn Asp Thr Thr Phe Tyr Glu Cys Glu Thr Gln Asn Pro Val Ser
   385 390 395 400
   Val Arg Arg Ser Asp Pro Val Thr Leu Asn Val Leu Tyr Gly Pro Asp
   405 410 415
   Ala Pro Thr Ile Ser Pro Leu Asn Thr Pro Tyr Arg Ala Gly Glu Asn
   420 425 430
   Leu Asn Leu Ser Cys His Ala Ala Ser Asn Pro Ala Ala Gln Tyr Phe
   435 440 445
   Trp Phe Val Asn Gly Thr Phe Gln Gln Ser Thr Gln Glu Leu Phe Ile
   450 455 460
   Pro Asn Ile Thr Val Asn Asn Ser Gly Ser Tyr Met Cys Gln Ala His
   465 470 475 480
   Asn Ser Ala Thr Gly Leu Asn Arg Thr Thr Val Thr Ala Ile Thr Val
   485 490 495
   Tyr Val Glu Leu Pro Lys Pro Tyr Ile Ser Ser Asn Asn Ser Asn Pro
   500 505 510
   Ile Glu Asp Lys Asp Ala Val Thr Leu Thr Cys Glu Pro Val Ala Glu
   515 520 525
   Asn Thr Thr Tyr Leu Trp Trp Val Asn Asn Gln Ser Leu Ser Val Ser
   530 535 540
   Pro Arg Leu Gln Leu Ser Asn Gly Asn Arg Ile Leu Thr Leu Leu Ser
   545 550 555 560
   Val Thr Arg Asn Asp Thr Gly Pro Tyr Glu Cys Gly Ile Gln Asn Ser
   565 570 575
   Glu Ser Ala Lys Arg Ser Asp Pro Val Thr Leu Asn Val Thr Tyr Gly
   580 585 590
   Pro Asp Thr Pro Ile Ile Ser Pro Pro Asp Leu Ser Tyr Arg Ser Gly
   595 600 605
   Ala Asn Leu Asn Leu Ser Cys His Ser Asp Ser Asn Pro Ser Pro Gln
   610 615 620
   Tyr Ser Trp Leu Ile Asn Gly Thr Leu Arg Gln His Thr Gln Val Leu
   625 630 635 640
   Phe Ile Ser Lys Ile Thr Ser Asn Asn Asn Gly Ala Tyr Ala Cys Phe
   645 650 655
   Val Ser Asn Leu Ala Thr Gly Arg Asn Asn Ser Ile Val Lys Asn Ile
   660 665 670
   Ser Val Ser Ser Gly Asp Ser Ala Pro Gly Ser Ser Gly Leu Ser Ala
   675 680 685
   Arg Ala Thr Val Gly Ile Ile Ile Gly Met Leu Val Gly Val Ala Leu
   690 695 700
   Met
   705
<210> 9
   <211> 81
   <212> DNA
   <213> Artificial Sequence
<220>
   <223> consensus sequence
<400> 9
   agcgttcctg gagcccaagc tcttctccac agaggaggac agagcaggca gcagagacca 60
   tggggccccc ctcagcccct c 81
<210> 10
   <211> 81
   <212> DNA
   <213> Homo sapiens
<400> 10
   aacgttcctg gaactcaagc tcttctccac agaggaggac agagcagaca gcagagacca 60
   tggagtctcc ctcggcccct c 81
<210> 11
   <211> 80
   <212> DNA
   <213> Homo sapiens
<400> 11
   agcattcctg gagctcaagc tctctacaaa gaggtggaca gagaagacag cagagaccat 60
   gggacccccc tcagcccctc 80
<210> 12
   <211> 81
   <212> DNA
   <213> Homo sapiens
<400> 12
   agcgttcctg gagcccaagc tctcctccac aggtgaagac agggccagca ggagacacca 60
   tggggcacct ctcagcccca c 81
<210> 13
   <211> 54
   <212> DNA
   <213> Homo sapiens
<400> 13
   gcacagagga gaacacgcag gcagcagaga ccatggggcc catctcagcc cctt 54
<210> 14
   <211> 78
   <212> DNA
   <213> Homo sapiens
<400> 14
   agagttcctg gagccccaag ctcttctcca cagaggacaa gcaggcagca gagaccatgg 60
   gttccccttc agcctgtc 78
<210> 15
   <211> 76
   <212> DNA
   <213> Homo sapiens
<400> 15
   tcctggagcc caggctcttt tccacagagg aggaaagagc aggcagcaga gaccatgggg 60
   cccccctcag cccctc 76
<210> 16
   <211> 76
   <212> DNA
   <213> Homo sapiens
<400> 16
   agcgttcctg gagcccagct cctctccaca gaccacaagc acccagcaga gaccatgggc 60
   cccccctcag ccgctc 76